# EUROPEAN PATENT APPLICATION

(11) **EP 4 834 206 A1**
(43) Date of publication of application: **04.11.2026**
(21) Application number: 26174883.4
(22) Date of filing: 27.04.2026
(51) Int. Cl.: A61B 5/00, A61B 34/20, A61B 34/00

(54) **INTRAOPERATIVE GRAPHICAL FEEDBACK FOR COCHLEAR IMPLANT PROCEDURES**

(30) Priority: 02.05.2025 US 202563799215 P; 09.01.2026 US 202663957251 P
(71) Applicant: Iotamotion, Inc., St. Paul, Minnesota 55110 (US); Nikou, Constantinos, Monroeville, Pennsylvania 15146 (US); Hansen, Marlan, Solon, Iowa 52333 (US); Scheperle, Rachel, Iowa City, IA 52240 (US)
(72) Inventor: NIKOU, Constantinos, Monroeville, Pennsylvania 15146 (US); HANSEN, Marlan, Solon, Iowa 52333 (US); SCHEPERLE, Rachel, Iowa City, Iowa 52240 (US)
(74) Representative: Murgitroyd & Company

(57) **Abstract**

A system for monitoring cochlear implant insertion can include a sensor configured to detect electrophysiologic responses at multiple frequencies. The sensor can include one or more recording electrodes configured to detect electrophysiologic responses generated by a cochlea of a patient in response to acoustic stimuli delivered at the multiple frequencies. The system can include a processor with memory storing instructions. When executed, the instructions can configure the processor to receive the electrophysiologic responses from the sensor and determine position data of a cochlear implant electrode array within an ear of the patient. The processor can generate a visual representation in real-time as the electrode array is inserted, using the electrophysiologic responses and the position data. The visual representation can be transmitted to a display and can include the electrophysiologic responses at the multiple frequencies plotted against an insertion angle, along with a current position indicator for the electrode array.

## Description

### CLAIM OF PRIORITY

This patent application claims the benefit of priority to Constantinos Nikou, U.S. Patent Application Serial Number 63/799,215, entitled "INTRAOPERATIVE GRAPHICAL FEEDBACK FOR COCHLEAR IMPLANT PROCEDURES," filed on May 2, 2025, (Attorney Docket No. 4700.013PRV) and to Constantinos Nikou, U.S. Patent Application Serial Number 63/957,251, entitled "INTRAOPERATIVE GRAPHICAL FEEDBACK FOR COCHLEAR IMPLANT PROCEDURES," filed on January 9, 2026, (Attorney Docket No. 4700.013PV2).

### STATEMENT REGARDING FEDERALLY SPONSORED RESEARCH OR DEVELOPMENT

This invention was made with government support under award number R44DC017640 awarded by the Department of Health and Human Services. The government has certain rights in this invention.

### TECHNICAL FIELD

Examples described herein generally relate to graphical feedback and, more specifically, to intraoperative graphical feedback for cochlear implant procedures.

### BACKGROUND

The cochlea is the auditory portion of the inner ear. The cochlea includes a spiraled, fluid-filled, conical chamber of bone in which waves propagate. The middle ear transduces sound waves into mechanical energy, vibrating the perilymph/endolymph, and the organ of Corti atop the basilar membrane, which contains the sensory hair cells that convert the vibrations to neurotransmitters that excite the cochlear nerve. The hair cells and nerves in the basal or outer region of the spiraled cochlea are more sensitive to higher frequencies of sound and are frequently the first part of the cochlea to lose sensitivity. The apical or inner region of the spiraled cochlea is more sensitive to lower frequencies.

Moderate to profound hearing loss affects a large number of people worldwide and can have a significant impact on a patient's physical and mental health, education, employment, and overall quality of life. Hearing loss can be caused by partial damage to the cochlea. Many patients with various degrees of hearing loss have partial damage to the cochlea in the high-frequency regions (basal cochlea) from common causes such as noise exposure, drugs, genetic mutations, or aging, but can retain adequate low-frequency hearing.

Cochlear implants have been used to treat patients with hearing loss. A cochlear implant is a medical device that can include an external processor, a subcutaneously implantable stimulator, and an electrode assembly sized and shaped for cochlear insertion. The processor microphones can convert sound signals into electrical signals, which are transmitted to the implantable stimulator. The physical properties (e.g., frequencies and amplitudes) of the acoustic signals are used within the processing strategy to determine how features of the electrical impulses are delivered by the electrode array surgically inserted into the cochlea. For instance, higher frequencies can result in greater stimulation from electrodes located at the outer or basal cochlear region, and lower frequencies can result in greater stimulation at a more inner or apical cochlear region.

Many patients who have lost high-frequency hearing but who have substantial residual, low-frequency hearing function in the apical cochlea, have significant difficulty with word understanding. A cochlear implant surgery can be performed by a surgeon to manually insert the electrode assembly into the damaged portion of a patient's cochlea (e.g., basal cochlea), while avoiding or minimizing any trauma to the undamaged cochlear regions to preserve the low-frequency hearing function. The cochlear implant can be used together with a hearing aid that acoustically stimulates the undamaged low-frequency sensitive apical cochlea.

### SUMMARY

The invention is a system, method and computer-readable medium as defined in the appended claims.

### BRIEF DESCRIPTION OF THE DRAWINGS

Various examples are illustrated in the figures of the accompanying drawings. Such examples are demonstrative and not intended to be exhaustive or exclusive examples of the present subject matter.
FIG. 1 illustrates an example of a robotically assisted implantation system and portions of an environment in which the intraoperative graphical feedback for cochlear implant procedures can operate.
FIG. 2 illustrates an example of a graphical representation for intraoperative graphical feedback for cochlear implant procedures.
FIG. 3 illustrates a block diagram of an example of an environment including a system for intraoperative graphical feedback for cochlear implant procedures.
FIG. 4 illustrates an example of a graphical representation including clearance indicators for intraoperative graphical feedback for cochlear implant procedures.
FIG. 5 illustrates an example of a graphical representation including bidirectional insertion feedback for intraoperative graphical feedback for cochlear implant procedures.
FIG. 6 illustrates a diagram of an example of a synchronization process for aligning reference frames for intraoperative graphical feedback for cochlear implant procedures.
FIG. 7 illustrates a block diagram of an example of a method.
FIG. 8 illustrates a block diagram of an example of a method.
FIG. 9 illustrates an example of a graphical representation including intraoperative graphical feedback for cochlear implant procedures.

### DETAILED DESCRIPTION

During cochlear implant insertion, a clinician can monitor the condition of the cochlea using electrocochleography (ECochG). ECochG measures electrical responses generated by the cochlea in response to acoustic stimulation at one or more frequencies. Changes in ECochG responses during insertion, such as drops in response amplitude, can indicate mechanical interaction between the electrode array and cochlear structures, including the basilar membrane.

Conventional ECochG monitoring systems display electrophysiologic responses as a function of time. As the insertion progresses, new response samples are appended to a time-series plot that advances along a time axis. This time-domain representation does not directly indicate where the electrode array is located within the cochlea at the time a given response was recorded. A clinician viewing a time-domain ECochG plot cannot determine, from the plot alone, the anatomical position at which a response change occurred.

Separately, the anatomy of the cochlea constrains the insertion. The scala tympani, through which the electrode array is advanced, tapers as it spirals from the base toward the apex. At some depth, the cross-sectional area of the scala tympani approaches or becomes smaller than the cross-sectional area of the electrode array. Beyond that depth, the electrode array can contact the basilar membrane or other cochlear structures. The depth at which this transition occurs varies among patients and depends on the geometry of the electrode array. Pre-operative imaging can provide estimates of the cochlear dimensions at various insertion angles, but this information is typically not presented to the clinician in a format that is spatially correlated with ECochG data during the procedure.

A further complication arises from the use of robotic insertion systems that can both advance and withdraw the electrode array. Robotic systems can reverse the electrode array to reposition it, but a time-domain ECochG display does not distinguish between responses recorded during insertion and responses recorded during withdrawal. The time-domain display also does not indicate the spatial relationship between a current electrode position and previously recorded response locations when the electrode is moving in reverse.

Additionally, the robotic system, the ECochG recording system, and the anatomical insertion angle operate in three independent reference frames. The robotic system tracks position in terms of motor encoder counts or linear displacement from a mechanical starting point. The ECochG system tracks responses by sample index from the time recording began. The anatomical insertion angle is measured relative to a landmark in the cochlea, such as the round window. These three reference frames do not inherently share a common origin. The robot may begin driving the electrode before or after the electrode tip reaches the round window. The ECochG system may begin recording before or after the electrode enters the cochlea. Without a defined synchronization event that aligns these three reference frames to a common anatomical starting point, the system cannot map a given ECochG sample to the insertion angle at which it was collected.

The present disclosure describes a system and method that addresses at least these issues. The system plots ECochG responses at multiple frequencies against insertion angle rather than time, spatially referencing each response to a position within the cochlea. The system overlays the plotted responses on a patient-specific cochlear model that includes cross-sectional area information, and displays a clearance indicator showing the available space between the electrode array and the cochlear wall at each insertion angle. The system includes a synchronization mechanism that aligns the robotic position, the ECochG sample index, and the anatomical insertion angle to a common starting point established by the clinician. The system supports bidirectional feedback, retaining insertion-phase responses at their recorded positions during withdrawal and visually distinguishing withdrawal-phase responses from insertion-phase responses. The combined display permits a clinician to correlate ECochG response changes with specific anatomical locations and with the available clearance at those locations during the procedure.

In examples, the present disclosure combines electrocochleography (ECochG) information with cochlear anatomy and insertion progress in a single display. The system plots ECochG responses at multiple frequencies (1000 Hz, 500 Hz, 250 Hz). The present disclosure can correlate the ECochG responses at multiple frequencies with the insertion angle and the current position of the implant within the ear. The graphical representation can include "ceiling height" information, represented by color-coded regions, showing the space available above the electrode during insertion. The color-coded regions can permit surgeons to correlate ECochG response drops with specific locations of potential trauma and available space within the cochlea in real-time.

The present disclosure can accommodate reversible insertions, providing continuous positional feedback relative to the anatomy of the ear. The present disclosure also relates to a method for synchronizing ECochG and insertion angle data with a defined starting point. The synchronization of the ECochG and insertion angle data with a defined starting point can increase precision in tracking the insertion process.

In examples, the system can also be used to display elements of scalar anatomy, depth of insertion, and fit of the electrode array relative to a position of the electrode array within the anatomy.

The system can integrate with robotic control systems to enable automated or assisted insertion procedures. By offering real-time, multi-factor analysis during the insertion process, this system can improve surgical outcomes and enable more precise, individualized implant placements.

The above discussion is intended to provide an overview of the subject matter of the present patent application. It is not intended to provide an exclusive or exhaustive explanation of the invention. The description below is included to provide further information about the present patent application.

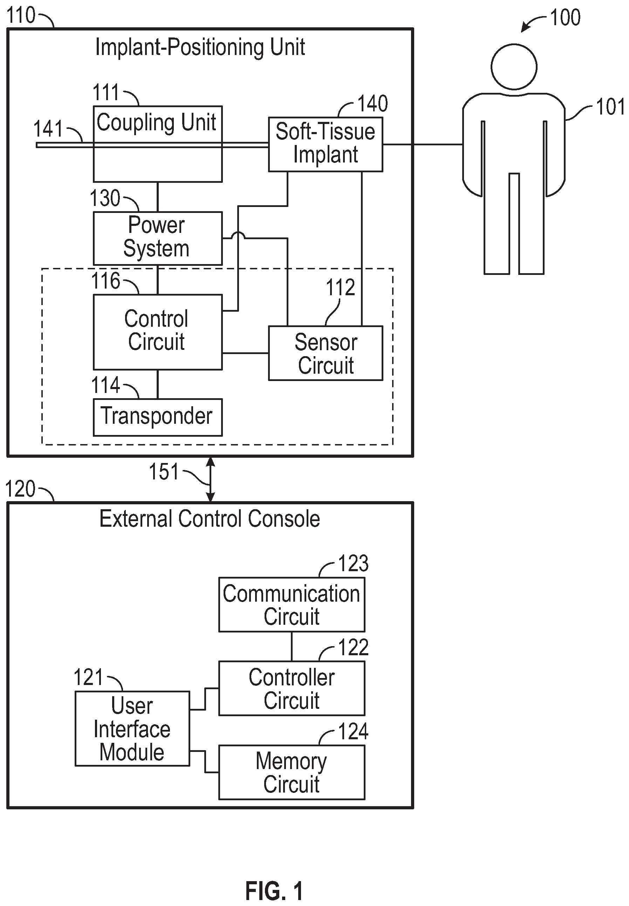

FIG. 1 is a diagram illustrating, by way of example and not limitation, a robotically assisted implantation system 100 and portions of an environment in which the system 100 can operate. The robotically assisted implantation system 100 can engage an implant 140, and robotically deliver and position the implant 140 into a target implantation site of a patient 101.

The system 100 can include an implant-positioning unit 110 and an external control console 120. The implant-positioning unit 110 can be an external device, or a completely or partially implantable device. The implant-positioning unit 110 can include one or more of a coupling unit 111, a sensor circuit 112, a power system 130, a transponder 114, and a control circuit 116. The coupling unit 111 can interface with an elongate member 141. An implant 140 can be coupled to the elongate member 141, such as on a distal end thereof. The coupling unit 111 includes actuating members arranged to engage at least a portion of the elongate member 141, and robotically propel the elongate member 141 to move the implant 140 into a target site of a patient 101. Examples of the actuating members can include motorized actuation via rollers, screws, gears, or rack-pinion, among others. In an example, the elongate member 141 can be an integral part of the implant 140, such as a tubular implant body or an elongate or telescoping shaft. Examples of such an implant can include an implantable lead or catheter. Alternatively, the elongate member 141 can be a part of a delivery system detachably coupled to the implant 140. Examples of such an implant can include a guidewire or an introducer that can snatch an implant at a particular location, such as at a distal portion of the elongate member 141.

The coupling unit 111 can frictionally move the elongate member 141 in a specific direction (e.g., forward for implant insertion, or reverse for implant extraction), at a specific rate, or for a specific distance relative to a reference point, such as the interface between the coupling unit 111 and the elongate member 141. Examples of the coupling unit 111 can include a leadscrew, a clamp, a set of rotors, or a rack and pinion arrangement, among other coupling mechanisms. The coupling unit 111 can compress against at least a portion of the elongate member 141 to produce sufficient friction between the coupling unit 111 and the elongate member 141. In some examples, the coupling unit 111 can include adjustable couplers for reversible or interchangeable connection between the implant-positioning unit 110 and the elongate member 141. In the event of implant exchange or replacement, the coupling unit 111 can operatively release the compression on the elongate member 141, which can then be removed from the implant-positioning unit 110. A new implant with an elongate member can be reloaded and engaged into the implant-positioning unit 110. The implant-positioning unit 110 need not be removed and can remain in place during implant replacement.

In some examples, the implant 140 can be delivered through a guide sheath. In some examples, the implant-positioning unit 110 includes separate structures to control a guide sheath separately from the implant 140. In other examples, the guide sheath can be positioned initially by the implant-positioning unit 110, and the implant 140 can be implanted through the previously positioned guide sheath.

In an example, the implant 140 can be a cochlear implant for treating hearing loss through electrostimulation of a specified cochlear region. The cochlear implant can include an implantable stimulator that can be implanted under the scalp, can generate electrical impulses, and deliver the electrical impulses to the electrode array through conductors in the elongate member 141. The electrode array can be surgically inserted into and positioned at the target cochlear site. In patients with impaired high-frequency hearing function but preserved low-frequency hearing function, a short electrode array of the implant can be positioned at the outer or basal cochlear region to deliver electrostimulation therein to restore high-frequency hearing function. The implant-positioning unit 110 can be a compact, lightweight micromechanical device suitable for direct attachment to the patient, such as on the patient's head during a cochlear implant surgery, while maintaining sufficient stability during the implantation. The implant-positioning unit 110 can be sized and shaped to facilitate patient attachment. In an example, the implant-positioning unit 110 can have a curved exterior contact surface that conforms to the contour of a body part of the patient 101, such as a head region.

Once the implant 140 has been positioned at the target site, the elongate member 141 can be disengaged from the implant 140. Alternatively, the elongate member 141 can remain attached to the implant 140 following the implantation. This allows a surgeon to re-optimize the implant position in an implant revision procedure following the initial implantation without the need for a surgery to reattach the implant 140 to the elongate member 141.

The power system 130 is configured to provide driving force to the coupling unit 111. The power system 130 includes a motor that can generate driving force and motion, and a power transmission unit to transmit the driving force and motion to the coupling unit 111 to actuate the motion of the elongate member 141. Examples of the motor can include stepper motors (e.g., micro- or nano-stepper motors), direct current (DC) motors, pneumatic or piezoelectric motors, ultrasonic motors, or linear motors, among others. The motor can be electrically coupled to a power source. In an example, the power source can include a rechargeable power source, such as a rechargeable battery or a supercapacitor. The rechargeable power source can be charged wirelessly by a portable device, such as a handheld device with circuitry configured to transfer energy to the rechargeable power source through electromagnetic induction or other transcutaneous powering means.

In the example as illustrated in FIG. 1, the power system 130 is at least partially included in or associated with the implant-positioning unit 110. Alternatively, the power system 130 can be at least partially included in or associated with the external control console 120. In another example, the power system 130 can be separated from the implant-positioning unit 110 and the external control console 120 and coupled to the coupling unit 111 via a connection. The connection can be a part of the transmission unit.

The control circuit 116 can be coupled to the transponder 114 to receive a motion control signal from the external control console 120. In an example, the coupling between the control circuit 116 and the transponder 114 is a wireless coupling. The motion control signal can specify values for various implant motion parameters and can be generated according to user programming instructions, such as those provided via the user interface module 121. The control circuit 116 can control the motor to generate driving force and motion according to the received motion control signal, and drive motion of the elongate member 141 via the power transmission unit and the coupling unit 111. Examples of the power transmission unit can include chains, spur gears, helical gears, planetary gears, or a gearhead, worm gears, miniature pulleys, shaft couplings, or timing belts, among others. The power transmission unit can adjust the speed or torque output from the motor, and deliver specific output to the coupling unit 111.

In various examples, the power system 130 can include two or more motors coupled to respective power transmission units, and the power transmission units are coupled to respective coupling units that engage the same elongate member 141 at different locations thereof. The two or more motors can be of the same or different types. The transponder 114 can receive from the external control console 120 a motion control signal for controlling each of the two or more motors. In an example, a user can program and control each of the motors independently, such as via the user interface module 121. The motion control signal specifies the configuration of, and input voltage or current to, each of the motors. According to the motion control signal, the control circuit 116 can control the two or more motors to generate respective torque, speed, or rotation direction. Through the elongate member 141, the implant-positioning unit 110 can operatively move the implant 140 in multiple axes and planes with up to six degrees of freedom (medial, lateral, superior, inferior, anterior, and posterior). In an example, a first motor produces a translational motion of the elongate member 141, and a second motor can produce a rotational motion of the elongate member 141. The control circuit 116 controls various translational motion parameters (e.g., translational rate, direction (advancement or withdrawal), distance relative to a reference point, a position of a distal end of the elongate member 141, an amount of axial force applied to the elongate member 141), and rotational motion parameters (e.g., angular position, angular displacement, angular velocity, or an amount of lateral or rotational force applied to the elongate member 141).

In some examples, the implant 140 can be attached to two or more elongate members, each representing an embodiment of the elongate member 141. Each elongate member can be coupled to a respective coupling unit representing an embodiment of the coupling unit 111. The transponder 114 can receive from the external control console 120 a motion control signal for controlling each of the two or more motors. According to the motion control signal, the control circuit 116 can control the two or more motors to generate driving forces to independently move the respective elongate members in different directions (e.g., advancement or withdrawal) or at different rates. Through independent control of multiple elongate members, the implant-positioning unit 110 can operatively move the implant 140 in multiple axes and planes. For example, the implant 140 can not only be advanced or withdrawn translationally, but can slant or rotate at different angles, thereby manipulating the target soft tissue at a desired position or with a desired shape.

In various examples, the control circuit 116 can change the shape or physical dimension of at least a portion of the implant 140, such as the topography of an implant surface interfacing with the target soft tissue. The implant 140 can include an array of micro-actuators on the tissue-interfacing surface of the implant. In response to an implant contour control signal from the external control console 120, the control circuit 116 can activate the micro-actuators to change the tissue-contacting surface contour. The change in the implant shape can result in changes in position or shape of at least a portion of the soft tissue. Compared to the motion control of the implant 140 via the power system 130 and the elongate member 141 for "macro position adjustment" of the target soft tissue, the surface contour control of the implant 140 via the micro-actuators can be used for "micro position adjustment" of the target soft tissue.

The sensor circuit 112 can be configured to sense information about the position or motion of the implant during implantation. The sensor circuit 112 can be attached to the motor or the power transmission unit within the power system 130, or associated with the coupling unit 111, to detect information about the position of the implant. Examples of the sensor circuit 112 can include a Hall-effect sensor integrated in the motor, one or more optical sensors attached to the coupling unit, a capacitive sensor configured to detect implant motion. The sensor circuit 112 can include force sensors included in the power system 130 or the coupling unit 111, or associated with the implant 140, to sense a parameter indicative of force or friction imposed on the implant during the implant advancement, such as axial, lateral, or radial forces when the implant 140 interacts with the target soft tissue. Examples of the force sensors can include resistors, capacitive sensors, piezoelectric material, or a strain gauge, among others. In an example, the force can be indirectly sensed by measuring the current supplied to the motor.

The current measurement can be transmitted to the external control console 120, where it is converted to the force (or torque) using the torque-current curve predetermined and stored in the memory circuit 124. In some examples, the sensor circuit 112 can include sensors on the implant 140 to provide information indicative of the shape or contour of the tissue-contacting surface of the implant 140, such as before and after applying voltage to the micro-actuators on the tissue-contacting surface of the implant.

The information acquired by the sensor circuit 112 can be forwarded to the external control console 120 via the communication link 151. The sensor information can be displayed or otherwise presented in a specific media format in the output module 126. In an example, the implant-positioning unit 110 can include an indicator to produce a visual or audio notification in response to the sensed sensor signal satisfying a specific condition. An indicator can include, for example, a light-emitting diode (LED) that can be turned on when the sensed sensor signal indicates the implant reaches the target site. In some examples, the indicator can include a plurality of LEDs with different colors or different pre-determined blinking patterns. The LED colors or the blinking patterns can correspond to various events encountered during the implantation procedure.

The implant-positioning unit 110 can include a fixation member to allow for detachable affixation of the implant-positioning unit 110 to the anchoring structure. The fixation can be invasive fixation that involves incision and/or penetration of the anchoring structure or the subcutaneous tissue. Examples of the fixation member can include one or more of a screw, a pin, a nail, a wire, a hook, a barb, a helix, a suture, a glue, or a magnet within the implant-positioning unit 110 coupled to one or more magnetic screws or pins affixed to the body part of the patient 101. In an example, the fixation member can include one or more of self-drilling screws, self-tapping screws, or self-piercing screws, such that no pilot hole needs to be drilled at the affixation site prior to screw installation.

In some examples, while some portion of the implant-positioning unit 110 is implantable, at least a portion of the implant-positioning unit 110 can be externally positioned, such as a portion of the power system 130 (e.g., power source or motor), the sensor circuit 112, or the transponder 114, among others. The non-implantable components can be packaged and affixed to the skin of a body part using non-invasive fixation means, such as clamps, temporary glues, or other holding devices that prevent lateral motion relative to the patient 101. The external package can be compact and lightweight for direct attachment to the patient, while maintaining sufficient stability during implantation. The external package can be sized and shaped to facilitate patient attachment, such as having a curved exterior surface that conforms to the contour of a body part of the patient 101.

The contact surface of the implant-positioning unit 110 can be processed to improve stability during the implant advancement procedure. In an example, the implant-positioning unit 110 can have an exterior surface with a rough finish, such as ridges, corrugations, teeth, or other coarse surface textures. Additionally or alternatively, the implant-positioning unit 110 can have one or more gripping elements configured to frictionally bond the implant-positioning unit 110 to a body part of the patient 101, such as the anchoring structure for subcutaneous implantation or epicutaneous placement. The gripping elements can be distributed on a portion of the exterior surface. Examples of the gripping elements can include penetrators, such as spikes, pins, or barbs protruding from the exterior surface. When the implant-positioning unit 110 is pressed and held against the attachment region, the rough surface or the gripping elements can provide sufficient friction or gripping force to securely hold the implant-positioning unit 110 in place relative to the patient 101 during implantation advancement.

The external control console 120 can include a dedicated hardware/software system, such as a programmer, a remote server-based patient management system, or alternatively a system defined predominantly by a controller software running on a standard personal computer. The external control console 120 can robotically control the coupling unit 111 to propel the elongate member 141 at a specific rate, in a specific direction, for a specific distance, or at a specific maximum force, thereby positioning the implant 140 at the target site of the patient 101. The external control console 120 can additionally receive information acquired by the sensor circuit 112. The external control console 120 can also receive measurement data from external systems that can be directly related to implant position. The external control console 120 can utilize such measurement data (e.g., physiological measurements) for closed-loop control of implant positioning and manipulation. For example, the external control console 120 can receive patient voice input via the user interface module 121 as feedback to manipulate the implant 140. In various examples, the external control console 120 can include a physiologic sensor configured to sense a physiologic signal, such as respiration or muscular movement of the patient. The controller circuit 122 can determine dynamic motion control feedback and control the positioning and manipulation of the implant further using the sensed physiologic signal.

The external control console 120 can include a user interface module 121 and a controller circuit 122. The user interface module 121 can include a user input module and an output module. The user input module can be coupled to one or more input devices, such as a keyboard, on-screen keyboard, mouse, trackball, touchpad, touch-screen, or other pointing or navigating devices. In some examples, the user input module can be incorporated in a mobile device communicatively coupled to the external control console 120, such as a handheld device. The user input module can be configured to receive motion control instructions from a user. The motion control instructions can include one or more target motion parameters characterizing desired movement of the elongate member 141 of the implant. For example, the target motion parameters can define maximum values or value ranges of the motion parameters. Examples of the target motion parameters can include a target movement rate, a target movement direction or orientation, a target movement distance, a target position of a distal end of the elongate member, or a target amount of force imposed on the elongate member 141. The movement of the implant can be activated at intervals of a predetermined step size. In an example of implantation of a cochlear implant, the target movement rate is approximately at 100-micron intervals. In an example, the target movement distance is approximately 1-35 millimeters.

The user interface module 121 can allow a user to select from a menu of multiple implant delivery protocols, customize an existing implant delivery protocol, adjust one or more motion parameters, or switch to a different implant delivery protocol during the implant delivery procedure. The external control console 120 can include a memory circuit 124 for storing, among other things, motion control instructions. In an example, one of the delivery protocols can include use of intraoperative physiologic measures that can reflect immediate changes in soft-tissue mechanics and insertion trauma pre-, during-, and post-insertion of the implant 140.

The output module can generate a human-perceptible presentation of information about the implant delivery control, including the programmable motion control parameters, and the motion control instructions provided by the user. The presentation can include audio, visual, or other human-perceptible media formats, or a combination of different media formats. The output module 126 can include a display screen for displaying the information, or a printer for printing hard copies of the information. The information can be displayed in a table, a chart, a diagram, or any other types of textual, tabular, or graphical presentation formats. Additionally or alternatively, the output module 126 can include an audio indicator, such as in the form of beeps, alarms, simulated voice, or other sound indicators.

The output module 126 can also generate a presentation of data sensed by the sensor circuit 112, including data such as the current position and movement rate of the implant, the force or friction applied to the implant motion. This allows a surgeon to monitor the progress of the implantation and adjust the motion control as needed. The presentation can include visual or audible notifications with specified patterns corresponding to different types of events encountered during implantation. In an example, the output module 126 can include a visual indicator, such as a light-emitting diode (LED) or an on-screen indicator on the display screen. A specific LED color or a specific blinking pattern can signal to the user a successful positioning of the implant at the target site. A different LED color or a different blinking pattern can alert to an excessive force imposed on the implant due to unintended tissue resistance during the implant advancement. The output module 126 can additionally or alternatively include an audio indicator, such as a beep with a specific tone, a specific frequency, or a specific pattern (e.g., continuous, intermittent, pulsed, sweep-up, or sweep-down sound). In an example, a beep or an alarm with a specific tone or pattern can signal to the user successful positioning of the implant at the target site. A beep or an alarm with a different tone or different pattern can alert to an excessive force imposed on the implant. In an example, the beep or the alarm can go off continuously as the sensor senses the implant approaching the target site. The sound frequency or the pulse rate of the beep or the alarm can increase as the implant gets closer and finally reaches the target site. In an example, the frequency of the beep or the alarm can correspond to a rate of motion, such as sounding for every one millimeter of motion. Audible feedback on the motion parameters can be advantageous in that the surgeon can be notified in real time of the implantation status or events encountered without the need to look away from the surgical field. This can assist the surgeon with enhanced surgical precision and patient safety. In some examples, the audible or visual sensor feedback can signal to the user that the sensed implant position, motion, or force has exceeded the programmed target or maximum parameter values.

The controller circuit 122 can be configured to generate an implant motion control signal and/or an implant contour control signal for controlling the implant-positioning unit 110 to deliver, position, and manipulate the implant 140. Such control signals can be generated according to the motion control instructions provided by the user via the user input module 125. In accordance with the motion control signal, the control circuit 116 can control the power system 130 to regulate one or more motion parameters of the elongate member 141, such as a movement rate, a movement direction or orientation, a movement distance, a position of a distal end of the elongate member, or an amount of force imposed on the elongate member 141, among others. In some examples, the controller circuit 122 can generate multiple motion control signals that can be used to respectively control multiple motors configured to drive different modes of motion (e.g., translational or rotational motions) on the same elongate member 141, or to drive different elongate members, as discussed above. In some examples, the controller circuit 122 can control the motion of the elongate member 141 further according to information about a patient's medical history or disease state received via the user input module 125 or stored in the memory circuit 124. In an example of a cochlear implant, information about a patient's tonotopic hearing loss pattern is used. The tonotopic hearing loss pattern represents a spatial distribution of frequency sensitivity along the axis of the cochlea length. Based on the input of the patient's tonotopic hearing loss pattern, the controller circuit 122 can automatically program one or more motion parameters, such as the electrode array insertion distance. In accordance with the motion control signal, the control circuit 116 can activate an array of micro-actuators, such as by applying a voltage map to change the tissue-contacting surface contour, thereby causing changes in shape or position of the target soft tissue.

The controller circuit 122 can remotely control the implant-positioning unit 110 via a communication circuit 123. The communication circuit 123 can transmit the motion control signal to the power system 130 via the communication link 151. The communication link 151 can include a wired connection, including a universal serial bus (USB) connection, or otherwise cables connecting the communication interfaces on the external control console 120 and the power system 130. The communication link 151 can alternatively include a wireless connection, such as a Bluetooth protocol, a Bluetooth low energy protocol, a near-field communication (NFC) protocol, Ethernet, IEEE 802.11 wireless, an inductive telemetry link, or a radiofrequency telemetry link, among others.

In various examples, the implant-positioning unit 110 can include a manual control mechanism in addition to the robotic control of the coupling unit 111. The manual control mechanism can bypass or override the robotic motion control of the implant 140. Examples of the manual control mechanism can include a dial turn, a screw, or a direct insertion technique. The output module 126 can enable a user to selectively enable a robotic mode for robotically assisted motion control via the power system 130, or a manual override mode for manual motion control of the elongate member 141. Alternatively, an operation on the manual control mechanism can automatically withhold or disable the robotic motion control of the elongate member 141.

Portions of the external control console 120 can be implemented using hardware, software, firmware, or combinations thereof. Portions of the external control console 120 can be implemented using an application-specific circuit that can be constructed or configured to perform one or more particular functions, or can be implemented using a general-purpose circuit that can be programmed or otherwise configured to perform one or more particular functions. Such a general-purpose circuit can include a microprocessor or a portion thereof, a microcontroller or a portion thereof, or a programmable logic circuit, or a portion thereof. For example, a "comparator" can include, among other things, an electronic circuit comparator that can be constructed to perform the specific function of a comparison between two signals, or the comparator can be implemented as a portion of a general-purpose circuit that can be driven by a code instructing a portion of the general-purpose circuit to perform a comparison between the two signals.

The system 100 can also include a cochlear implant insertion monitoring system as follows:

The sensor circuit 112 of the implant-positioning unit 110 can be configured to detect electrophysiologic responses generated by the cochlea in response to acoustic stimulation at multiple frequencies. In examples, the sensor circuit 112 can include an electrocochleography (ECochG) sensor for detecting ECochG responses at the multiple frequencies. The sensor circuit 112 can also be configured to determine position data indicative of a depth of insertion of the cochlear implant electrode array within the ear of the patient.

The sensor circuit 112, when configured to detect electrophysiologic responses, can include one or more recording electrodes and a signal conditioning circuit. The one or more recording electrodes can be located on the cochlear implant electrode array, such as on the most apical electrode contact, on a dedicated recording contact disposed on the electrode array, or on a separate extracochlear reference electrode, or on a combination thereof. In examples, the one or more recording electrodes can include an intracochlear recording electrode disposed on the electrode array and an extracochlear reference electrode positioned outside the cochlea, such as on a needle electrode placed through the tympanic membrane, on a surface electrode placed on the promontory, or on a ball electrode placed at the round window niche. In examples, the one or more recording electrodes can include one or more of the stimulation contacts of the cochlear implant electrode array that are repurposed for recording during the insertion procedure.

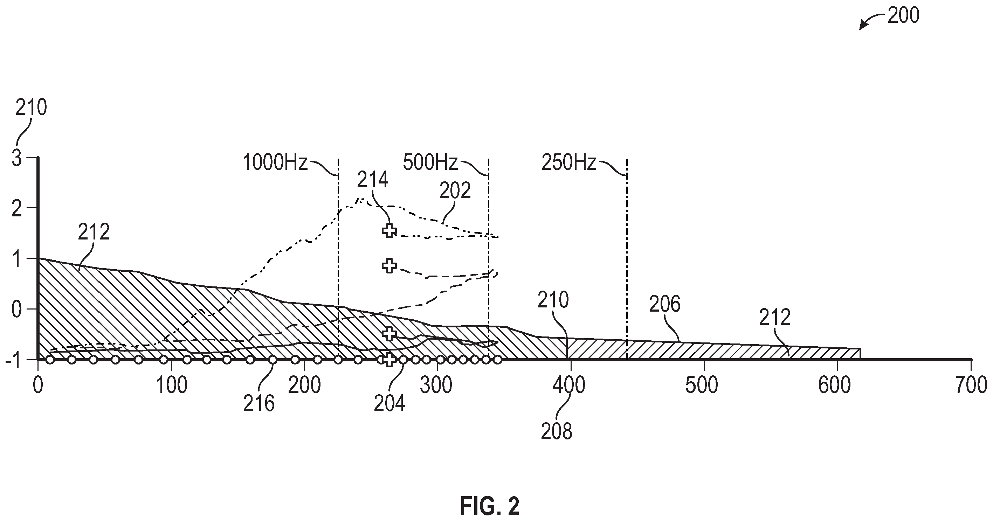

The signal conditioning circuit of the sensor circuit 112 can include an amplifier configured to amplify the electrophysiologic responses detected at the one or more recording electrodes, a filter configured to attenuate signal components outside a frequency band of interest, and an analog-to-digital converter configured to digitize the amplified and filtered electrophysiologic responses. The digitized electrophysiologic responses can be transmitted from the sensor circuit 112 to the controller circuit 122 via the communication link 151 for processing and inclusion in the visual representation 200 (FIG. 2). In examples, the signal conditioning circuit can be disposed within the implant-positioning unit 110. In other examples, the signal conditioning circuit can be disposed within the external control console 120 or in a separate recording unit communicatively coupled to the external control console 120.

The system 100 can further include an acoustic stimulus source configured to deliver acoustic stimuli to the ear of the patient at the multiple frequencies. The acoustic stimulus source can be separate from the sensor circuit 112. The acoustic stimulus source can include an insert earphone positioned in the ear canal of the patient, a circumaural or supra-aural headphone placed over the ear, a bone conduction transducer coupled to the skull of the patient, or a speaker positioned adjacent to the ear of the patient. The acoustic stimuli can include tone bursts, click stimuli, chirp stimuli, or continuous tones at each of the multiple frequencies. In examples, the multiple frequencies can include 250 Hz, 500 Hz, and 1000 Hz. In other examples, the multiple frequencies can include two frequencies, four or more frequencies, or a single frequency, depending on the clinical protocol. The acoustic stimulus source can be controlled by the controller circuit 122 or by a separate stimulus generator communicatively coupled to the controller circuit 122. The controller circuit 122 can coordinate the timing of the acoustic stimuli delivered by the acoustic stimulus source with the acquisition of the electrophysiologic responses by the sensor circuit 112, such that each stimulus presentation at a given frequency is paired with a corresponding response acquisition window.

The electrophysiologic responses detected by the sensor circuit 112 can include one or more of a cochlear microphonic (CM), a summating potential (SP), a compound action potential (CAP), or an auditory nerve neurophonic (ANN). The cochlear microphonic is an alternating-current voltage generated by the outer hair cells of the cochlea that mirrors the waveform of the acoustic stimulus. The summating potential is a direct-current shift in the baseline of the cochlear response that reflects the nonlinear transduction properties of the hair cells. The compound action potential is a synchronous response of the auditory nerve fibers to the onset of the acoustic stimulus. In examples, the amplitude plotted in the visual representation 200 (FIG. 2) can correspond to the amplitude of the cochlear microphonic at each of the multiple frequencies. In other examples, the plotted amplitude can correspond to the summating potential, the compound action potential, or a derived metric computed from a combination of one or more of these components. The selection of which component or components to plot can be configurable by the clinician via the user interface module 121.

The sensor circuit 112 can acquire electrophysiologic responses at a predetermined sampling interval. The sampling interval can be defined in terms of time, such as acquiring one response measurement every N milliseconds, where N can be in the range of about 100 milliseconds to about 5000 milliseconds. The sampling interval can alternatively be defined in terms of insertion distance, such as acquiring one response measurement every M micrometers of electrode array advancement, where M can be in the range of about 50 micrometers to about 500 micrometers. The sampling interval can alternatively be defined in terms of insertion angle, such as acquiring one response measurement every P degrees of angular advancement within the cochlea, where P can be in the range of about 1 degree to about 15 degrees. In examples, the electrode array can be paused during each response acquisition to reduce motion artifact in the recorded electrophysiologic response. In other examples, the electrode array can move continuously while the sensor circuit 112 acquires responses, and the controller circuit 122 can time-stamp each acquired response and correlate the time-stamp with the position data to associate each response with a corresponding insertion angle. The controller circuit 122 can average multiple response acquisitions at each sampling point to improve the signal-to-noise ratio. The number of averages can be configurable by the clinician via the user interface module 121, such as in the range of about 10 averages to about 500 averages per sampling point.

The external control console 120 can be equipped with the necessary memory and processing capabilities to function as the core of the monitoring system. The controller circuit 122 can be programmed to receive ECochG data from the ECochG sensor and angle data from the sensor circuit 112. Using this data, the controller circuit 122 can generate a visual representation of the insertion process in real-time. This visual representation, including ECochG responses plotted against insertion angle and a current position indicator for the electrode array, can be displayed on the output module 126.

The memory circuit 124 can store a cochlear model based on the ear of the patient. The controller circuit 122 can incorporate the cochlear model into the visual representation. The system can determine the diameter, e.g., a cross-sectional area, of the ear relative to the insertion angle using this model and calculate available space, e.g., a ceiling height, or a difference between a cross-sectional area of the cochlear implant and the cross-sectional area of the anatomy of the ear, for the electrode array. The output module 126 can display this information using color-coding to represent different degrees of available space, providing surgeons with crucial information about the spatial relationships within the cochlea during insertion.

The control circuit 116 in the implant-positioning unit 110 can be programmed to work in conjunction with the controller circuit 122 to enable robotic control of the insertion process based on the ECochG and angle data. This integration allows for potential automated adjustments to the insertion speed or trajectory based on the data, enhancing the precision and safety of the procedure.

Additionally, the system can be programmed to detect patterns in the ECochG responses that might indicate potential trauma during insertion. When such patterns are detected, the system can generate alert signals, displayed via the output module 126, to notify the surgical team of potential issues.

The system of the present disclosure can provide comprehensive monitoring and control of the cochlear implant insertion process. The system of the present disclosure can provide surgeons with enhanced visualization, precision, and safety features during the medical procedure.

More examples of the robotic insertion of a cochlear implant can be found in the works of the present applicant. For example, Patent Publication to Christopher Kaufmann, US Patent Application Number 62/872,625, entitled "MODULAR IMPLANT DELIVERY AND POSITIONING SYSTEM," filed on July 10, 2019, and assigned to IotaMotion, Inc., and all related applications and granted patents.

FIG. 2 illustrates an example of a graphical representation 200 for intraoperative graphical feedback for cochlear implant procedures. The graphical representation can include ECochG responses 202, position data 204, a cochlear model 206, depth of insertion 208, cross-sectional area dimension 210, and a clearance indicator 212. In examples, the insertion 208 can be on the x-axis and the cross-sectional area dimension 210 can be on the y-axis of the visual representation 200. The cross-sectional area dimension 210 can also be shown by a perimeter of the cochlear model 206.

The system 100 shown in FIG. 1 can generate the visual representation 200 illustrated in FIG. 2. The sensor circuit 112 of the implant-positioning unit 110 can detect electrophysiologic responses at multiple frequencies. An acoustic stimulus source can deliver acoustic stimuli at the multiple frequencies to the ear of the patient, and the sensor circuit 112 can detect the resulting electrophysiologic responses generated by the cochlea. For example, the sensor circuit 112 can include an electrocochleography (ECochG) sensor configured to detect ECochG responses, such as the cochlear microphonic, at each of the multiple frequencies. Herein, such responses can be discussed as the real-time ECochG responses 202. The sensor circuit 112 can also determine position data indicative of a depth of insertion of the elongate member 141 within the ear of the patient. In examples, the sensor circuit 112 can determine a position of the elongate member 141 within the ear, determine an insertion angle of the elongate member 141 or another portion of the implant 140, or the like. In examples, the position of the implant 140 or the elongate member 141 can also be calculated based on one or more predetermined or calculated insertion rates of the implant 140 or the elongate member 141 and an elapsed time inserting the implant 140 or the elongate member 141.

In examples, the implant-positioning unit can use dead-reckoning techniques, e.g., counting pulses in a stepper motor, or using a relative rotary encoder mounted to the motor shaft, to estimate the depth of insertion relative to a starting point. In examples, the clinician can determine the starting position of the implant. For example, once the implant is in a known position, the clinician can "zero point" or set a position of the implant. The system can then correlate the zero point position in images, models, or the like to indicate anatomy of the patient. For example, the clinician can set the zero point to the round window opening of the cochlea, and the starting point can be indicated on the model, visual representation, or a live image of the ear of the patient. The setting of this zero point could be triggered by an element on a graphical user interface, triggered by other events within the system, or could be remotely triggered by a connected system.

The controller circuit 122 can receive the ECochG responses 202 from the ECochG sensor and position data from the position sensor or calculate the position data of the implant 140 or the elongate member 141 based on known insertion parameters. The controller circuit 122 can synchronize the ECochG responses 202 and the position data 204 with a defined starting point of the cochlear implant insertion, increasing precision in tracking the insertion process and providing a consistent reference point for analysis and visualization. As discussed herein, the clinician can set the starting point of the cochlear implant insertion, and the system can overlay this position on one or more of the visual representation, the model, or a live image of the ear of the patient.

Using this synchronized data, the controller circuit 122 can generate the visual representation 200 as the implant 140 or the elongate member 141 is inserted into the patient's ear. The visual representation 200 can accommodate reversible insertions, providing continuous positional feedback relative to the anatomy of the ear during both insertion and withdrawal of the implant 140 or the elongate member 141. The continuous positional feedback relative to the anatomy of the ear can help a clinician reposition the implant 140 or the elongate member 141 within the ear of the patient.

The output module 126 can display the visual representation 200, which can include ECochG responses 202 at multiple frequencies plotted against the position data 204 for the implant 140 or the elongate member 141.

The memory circuit 124 can store a cochlear model 206 based on the ear of the patient. The cochlear model 206 can include detailed information about the cochlear anatomy, including the tonotopic regions of the cochlea. Tonotopic regions represent the spatial distribution of frequency sensitivity along the length of the cochlea, with different areas responding to different sound frequencies. The cochlear model 206 can be determined based on preoperative imaging, intraoperative imaging, machine learning, AI generation, or a combination thereof. The controller circuit 122 can include the cochlear model 206 in the visual representation 200. The controller circuit 122 can update the ECochG responses 202, the position data 204, and the cochlear model 206 based on any one or more of the ECochG responses 202, the position data 204, or any other data provided by components of the system 100. The update of the ECochG responses 202, the position data 204, and the cochlear model 206 can provide a clinician with live information intraoperatively to permit the clinician to adapt the medical procedure to the specific characteristics of the cochlea or other ear anatomy of the patient as revealed during the insertion process of the implant 140 or the elongate member 141.

Using this dynamic version of the visual representation 200, the controller circuit 122 can determine the cross-sectional area dimension 210 of the ear relative to the insertion depth and calculate the available space for the implant 140 or the elongate member 141. The controller circuit 122 can display the available space on the visual representation 200 as a clearance indicator 212 or can include visual updates based on the space available relative to the insertion depth of the implant 140 or the elongate member 141. For example, the controller circuit 122 can generate a color-coded representation of this available space, incorporating it into the visual representation 200 as the clearance indicator 212. The clearance indicator 212 can include different colors, patterns, numbers, or other indicia that can be indicative of different degrees of available space. For example, the clearance indicator 212 can indicate when there are no clearance concerns based on the cross-sectional area dimension 210 being larger than a major dimension of the implant 140 or the elongate member 141, the clearance indicator 212 can indicate when the cross-sectional area dimension 210 is greater than the major dimension of the implant 140 or the elongate member 141 by half the diameter of the implant 140 or the elongate member 141, and the clearance indicator 212 can indicate when the major diameter of the implant 140 or the elongate member 141 is equal to or less than the cross-sectional area dimension 210.

The controller circuit 122 can correlate changes in the ECochG responses 202 with specific locations, e.g., based on the position data 204, in the cochlear model 206. The controller circuit 122 can display the position data 204 as current position indicators 214 or past position indicators 216. Both of the current position indicators 214 and the past position indicators 216 of the position data 204 can be overlaid on the visual representation 200. The depth of insertion 208 can also be indicated in the visual representation 200, such as along the x-axis as a measured depth or an angle of insertion of the implant 140 or the elongate member 141. As discussed herein, the depth or angle of insertion can be calculated based on known parameters of the insertion of the implant 140 or the elongate member 141, or can be determined based on sensor signals, visual detection, or other position-determining means.

Additionally, the visual representation 200 can include a graphical indicator of insertion progress relative to predefined anatomical landmarks within the cochlea. These landmarks can be derived from the cochlear model 206 and can include structures such as the round window, the basal turn, and the apex of the cochlea.

Additionally, the visual representation could include a preoperative or intraoperative audiogram, which captures the thresholds of hearing for the patient. This audiogram may be overlaid along the same data, along the insertion depth axis, because the tonotopic map of the cochlear model can provide a translation from a preoperative hearing threshold frequency to a depth of insertion. This may be useful for the surgeon to appreciate, with a combined view, where anatomic limitations to insertion may exist, and whether the given electrode could be inserted deeply enough to provide meaningful support to hearing. Alternatively, the combined preoperative hearing thresholds could be used for a "partial" insertion of the electrode array-a strategy where anatomic limitations in the size of the scala tympani and/or existing acoustical hearinglevels at lower frequencies are sufficient to obviate the need for electrical stimulation in the apical region of the cochlea and therefore may not warrant a deep insertion.

The controller circuit 122 can generate a multi-factor analysis based on the ECochG data 202, the position data 204, and the cochlear model 206. The multi-factor analysis can include various factors simultaneously, such as insertion angle, changes in one or more of the ECochG responses 202, the position data 204, or the cochlear model 206, or the clearance indicator 212. The controller circuit 122 can include this multi-factor analysis in the visual representation 200, providing a comprehensive view of the insertion process.

The control circuit 116 can communicate with the controller circuit 122 to enable robotic control of the insertion process based on one or more of the ECochG responses 202, the position data 204, the cochlear model 206, or the clearance indicator 212. Therefore, the control circuit 116 can adjust insertion parameters, e.g., the insertion speed or trajectory of the implant 140 or the elongate member 141.

The controller circuit 122 can detect patterns in the ECochG responses 202, the position data 204, the cochlear model 206, the clearance indicator 212, or any other data collected by components of the system 100 to indicate potential trauma during insertion of the implant 140 or the elongate member 141. In response to a potential trauma, the control circuit 116 or the controller circuit 122 can generate alerts, signals, or other visual or audible indicia to notify the clinician of such a determination. Moreover, the control circuit 116 or the controller circuit 122 can alter the insertion of the implant 140 or the elongate member 141 based on a likelihood to cause trauma in the ear of the patient.

The system 100, as discussed in the present disclosure, can provide comprehensive monitoring and control of the cochlear implant insertion process, offering enhanced visualization, precision, and safety features during the procedure. By incorporating these advanced features, the system 100 can offer a more adaptable tool for monitoring and guiding cochlear implant insertions, potentially improving surgical outcomes and patient care.

In examples, the visual representation 200 can include additional information that can be helpful to a clinician during the insertion of the implant 140 or the elongate member 141. For example, an audiogram or a pure-tone hearing test, a tympanometry, an otoacoustic emissions test, an auditory brainstem response, or any other test related to the ear, cochlea, brain, or other anatomy of the patient, or the like. In examples, the visual representation 200 can organize each of these such that the information can be combined in a single visual representation 200 or the clinician can choose the data or information provided on the visual representation 200.

A method for monitoring cochlear implant insertion can be implemented using a controller that includes processing circuitry executing instructions stored in a memory. The controller can initialize the sensor circuit 112 to detect electrophysiologic responses at multiple predetermined frequencies, such as 1000 Hz, 500 Hz, and 250 Hz, and can control an acoustic stimulus source to deliver acoustic stimuli at the multiple predetermined frequencies to the ear of the patient. The controller can establish a starting point for the insertion process of a cochlear implant electrode array. The starting point can be a reference for subsequent measurements, e.g., the electrophysiologic responses or the insertion depth of the implant or the elongated member, or the like. As the insertion progresses, the controller can receive electroacoustic response data from the sensor and determine position data based on the known insertion rate and elapsed time since the starting point. In examples, the controller can receive a signal from an optical sensor, a position sensor, or the like, that can be indicative of a position of the implant or the elongated member in the ear of the patient.

Using the obtained data, the controller can generate and continuously update a visual representation. The representation can include electrophysiologic responses plotted against the depth of insertion or the insertion angle, derived from the position data, and a current position indicator for the electrode array. The controller can analyze the electroacoustic data to detect significant changes or anomalies, generating alert indicators when necessary. To enhance the visualization, the controller can incorporate a cochlear model based on pre-operative imaging of the patient's cochlea. The controller can overlay the plotted responses and position indicator onto this model, providing context for the insertion process.

The method can allow for further refinements and additional features. For instance, the controller can synchronize the electroacoustic data and position data with the defined starting point, increasing precision in tracking the insertion process. It can accommodate reversible insertions, providing feedback during both insertion and withdrawal of the electrode array. The controller can update the cochlear model based on the received data, adapting to the specific characteristics of the patient's cochlea. It can perform a multi-factor analysis considering various parameters such as insertion depth, response changes, and available space within the cochlea. The visual representation can include graphical indicators of insertion progress relative to predefined anatomical landmarks. In cases where the system includes a robotic insertion mechanism, the controller can adjust the insertion speed or trajectory based on the data. By implementing these features, the method can provide comprehensive monitoring and control of the cochlear implant insertion process, potentially improving surgical precision and patient outcomes. Various combinations of available information can be aligned to the anticipated location of the implant, as well as the full anatomy of the cochlea along the possible depths of insertion, can be included in a system, depending on the preferences of the user as well as the availability of information (such as one or more audiograms, or ECochG, or other biofeedback measurements).

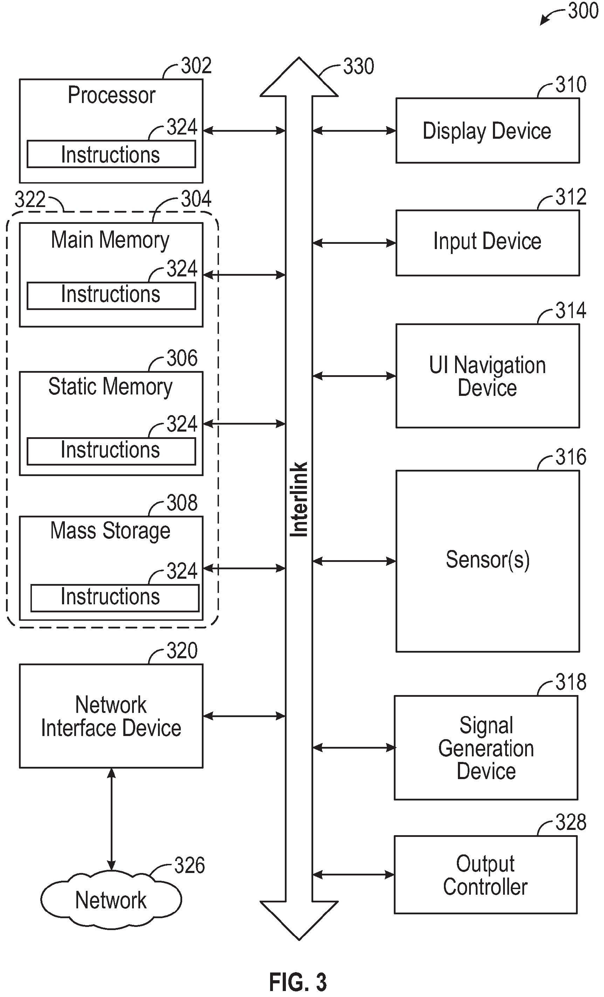

FIG. 3 illustrates a block diagram of an example machine 300 upon which any one or more of the techniques (e.g., methodologies) discussed herein may be performed. Examples, as described herein, may include, or may operate by, logic or a number of components, or mechanisms in the machine 300. Circuitry (e.g., processing circuitry) is a collection of circuits implemented in tangible entities of the machine 300 that include hardware (e.g., simple circuits, gates, logic, etc.). Circuitry membership may be flexible over time. Circuitries include members that may, alone or in combination, perform specified operations when operating. In an example, the hardware of the circuitry may be immutably designed to carry out a specific operation (e.g., hardwired). In an example, the hardware of the circuitry may include variably connected physical components (e.g., execution units, transistors, simple circuits, etc.), including a machine-readable medium physically modified (e.g., magnetically, electrically, moveable placement of invariant massed particles, etc.) to encode instructions for the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, in an example, the machine-readable medium elements are part of the circuitry or are communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time. Additional examples of these components with respect to the machine 300 follow.

In alternative embodiments, the machine 300 may operate as a standalone device or may be connected (e.g., networked) to other machines. In a networked deployment, the machine 300 may operate in the capacity of a server machine, a client machine, or both in server-client network environments. In an example, the machine 300 may act as a peer machine in peer-to-peer (P2P) (or other distributed) network environments. The machine 300 may be a personal computer (PC), a tablet PC, a set-top box (STB), a personal digital assistant (PDA), a mobile telephone, a web appliance, a network router, switch, or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), or other computer cluster configurations.

The machine (e.g., computer system) 300 may include a hardware processor 302 (e.g., a central processing unit (CPU), a graphics processing unit (GPU), a hardware processor core, or any combination thereof), a main memory 304, a static memory (e.g., memory or storage for firmware, microcode, a basic-input-output (BIOS), unified extensible firmware interface (UEFI), etc.) 306, and mass storage 308 (e.g., hard drive, tape drive, flash storage, or other block devices), some or all of which may communicate with each other via an interlink (e.g., bus) 330. The machine 300 may further include a display unit 310, an alphanumeric input device 312 (e.g., a keyboard), and a user interface (UI) navigation device 314 (e.g., a mouse). In an example, the display unit 310, input device 312, and UI navigation device 314 may be a touch screen display. The machine 300 may additionally include a storage device (e.g., drive unit) 308, a signal generation device 318 (e.g., a speaker), a network interface device 320, and one or more sensors 316, such as a global positioning system (GPS) sensor, compass, accelerometer, or other sensor. The machine 300 may include an output controller 328, such as a serial (e.g., universal serial bus (USB)), parallel, or other wired or wireless (e.g., infrared (IR), near field communication (NFC), etc.) connection to communicate or control one or more peripheral devices (e.g., a printer, card reader, etc.).

Registers of the processor 302, the main memory 304, the static memory 306, or the mass storage 308 may be, or include, a machine-readable medium 322 on which is stored one or more sets of data structures or instructions 324 (e.g., software) embodying or utilized by any one or more of the techniques or functions described herein. The instructions 324 may also reside, completely or at least partially, within any of the registers of the processor 302, the main memory 304, the static memory 306, or the mass storage 308 during execution thereof by the machine 300. In an example, one or any combination of the hardware processor 302, the main memory 304, the static memory 306, or the mass storage 308 may constitute the machine-readable media 322. While the machine-readable medium 322 is illustrated as a single medium, the term "machine-readable medium" may include a single medium or multiple media (e.g., a centralized or distributed database, and/or associated caches and servers) configured to store the one or more instructions 324.

The term "machine-readable medium" may include any medium that is capable of storing, encoding, or carrying instructions for execution by the machine 300 and that cause the machine 300 to perform any one or more of the techniques of the present disclosure, or that is capable of storing, encoding, or carrying data structures used by or associated with such instructions. Non-limiting machine-readable medium examples may include solid-state memories, optical media, magnetic media, and signals (e.g., radio frequency signals, other photon-based signals, sound signals, etc.). In an example, a machine-readable medium comprises a machine-readable medium with a plurality of particles having invariant (e.g., rest) mass, and thus are compositions of matter. Accordingly, non-transitory machine-readable media are machine-readable media that do not include transitory propagating signals. Specific examples of non-transitory machine-readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magnetooptical disks; and CD-ROM and DVD-ROM disks.

The instructions 324 may be further transmitted or received over a communications network 326 using a transmission medium via the network interface device 320 utilizing any one of a number of transfer protocols (e.g., frame relay, Internet Protocol (IP), Transmission Control Protocol (TCP), User Datagram Protocol (UDP), Hypertext Transfer Protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 320 may include one or more physical jacks (e.g., Ethernet, coaxial, or phone jacks) or one or more antennas to connect to the communications network 326. In an example, the network interface device 320 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding, or carrying instructions for execution by the machine 300, and includes digital or analog communications signals or other intangible medium to facilitate communication of such software. A transmission medium is a machine-readable medium.

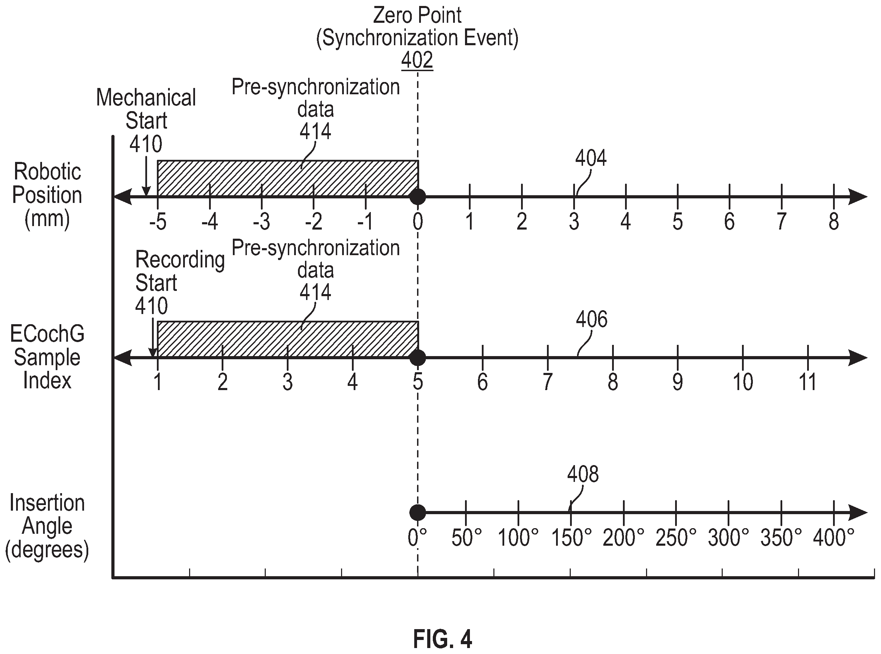

FIG. 4 illustrates an example of a synchronization diagram 400 showing the alignment of three independent reference frames used during cochlear implant insertion. The robotic position 404 of the implant-positioning unit 110 (FIG. 1), the ECochG sample index 406 of the sensor, and the insertion angle 408 within the ear of the patient can represent three independent reference frames that do not inherently share a common origin. The synchronization diagram 400 illustrates how the system can align these three reference frames to a single defined starting point, referred to as a zero point 402, to enable the generation of the visual representation 200 (FIG. 2).

The top number line represents the robotic position 404 measured in millimeters. The robotic position 404 can correspond to the linear displacement of the elongate member 141 (FIG. 1) as driven by the coupling unit 111 (FIG. 1). A mechanical start 410 can indicate the position of the elongate member 141 at the time the implant-positioning unit 110 begins driving the elongate member 141. As shown, the mechanical start 410 can be at an arbitrary position along the robotic position 404, such as at -5 mm, and does not necessarily correspond to the anatomical starting point of the insertion.

The middle number line represents the ECochG sample index 406. The ECochG sample index 406 can correspond to the sequential numbering of electroacoustic response samples acquired by the sensor. A recording start 412 can indicate the sample index at which the sensor begins recording. As shown, the recording start 412 can be at an arbitrary sample index, such as sample 1, that does not correspond to the anatomical starting point of the insertion.

The bottom number line represents the insertion angle 408 measured in degrees. The insertion angle 408 can correspond to the angular depth of the cochlear implant electrode array within the cochlea of the patient. The insertion angle 408 can begin at 0° at the zero point 402 and can increase as the electrode array advances into the cochlea.

A vertical dashed line represents the zero point 402. The zero point 402 can correspond to an anatomical reference location within the ear of the patient, such as the round window opening of the cochlea. At the zero point 402, the system can align all three reference frames by setting the robotic position 404 to correspond to 0° insertion angle 408, aligning the ECochG sample index 406 to the 0° position, and establishing the insertion angle 408 at 0°. A clinician can establish the zero point 402 by providing a user input, such as activating a control on the user interface module 121 (FIG. 1), indicating that the cochlear implant electrode array is positioned at the anatomical reference location. In other examples, the system can detect the anatomical reference location using sensor data, image registration, or a combination thereof.

Hatched regions labeled pre-synchronization data 414 can extend from the mechanical start 410 and the recording start 412 to the zero point 402 on the robotic position 404 and ECochG sample index 406 number lines, respectively. The pre-synchronization data 414 can represent data acquired before the zero point 402 was established. The system can discard the pre-synchronization data 414 or can retain the pre-synchronization data 414 for diagnostic purposes or for adjusting the cochlear model 206 (FIG. 2). In examples, the pre-synchronization data 414 is not included in the visual representation 200 plotted against the insertion angle 408.

Following synchronization at the zero point 402, each increment of robotic position 404 can correspond to a specific ECochG sample index 406 and a specific insertion angle 408. This alignment can permit the controller circuit 122 (FIG. 1) to map each electroacoustic response to a corresponding insertion angle and anatomical position within the cochlear model 206, enabling the generation of the visual representation 200 in which the electrophysiologic responses 202 (FIG. 2) can be plotted against the insertion angle derived from the position data 204 (FIG. 2).

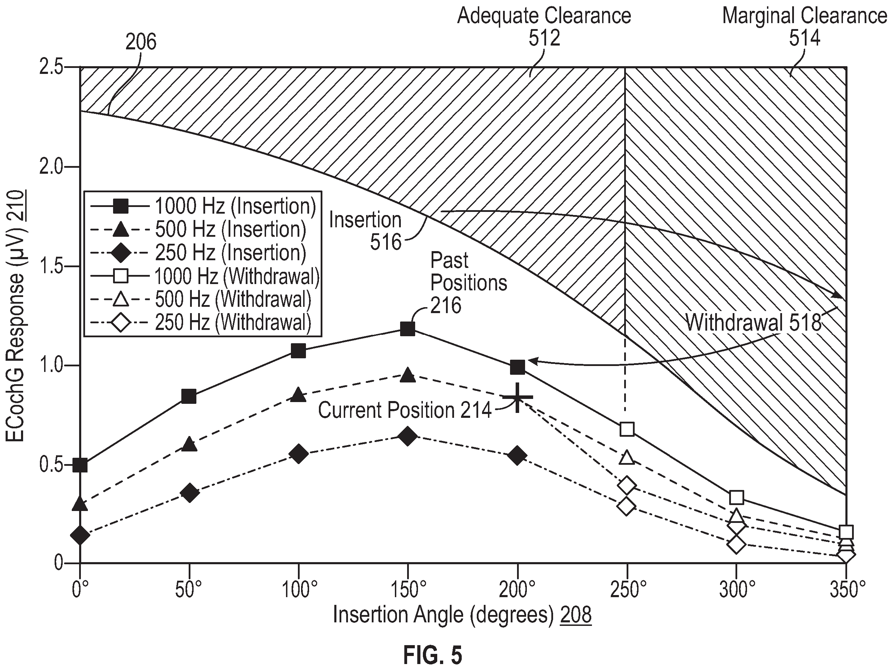

FIG. 5 illustrates an example of a graphical representation for bidirectional insertion feedback during a cochlear implant procedure. The graphical representation of FIG. 5 can depict the visual representation 200 (FIG. 2) during a withdrawal of the cochlear implant electrode array.

The graphical representation of FIG. 5 can include the cochlear model 206 (FIG. 2), the depth of insertion shown as insertion angle 208 (FIG. 2) in degrees on the x-axis, and the electroacoustic response amplitude shown in microvolts (µV) 210 (FIG. 2) on the y-axis. The graphical representation can further include an adequate clearance region 512, a marginal clearance region 514, an insertion direction indicator 516, a withdrawal direction indicator 518, a current position indicator 214 (FIG. 2), and past position indicators 216 (FIG. 2).

The cochlear model 206 can be represented as a curve that descends from a higher value at lower insertion angles to a lower value at higher insertion angles, reflecting the tapering of the scala tympani as the cochlea spirals inward. The adequate clearance region 512 and the marginal clearance region 514 can be displayed as hatched regions bounded by the cochlear model 206 and an upper border of the graphical representation. The adequate clearance region 512 can correspond to insertion angles where the cross-sectional area of the ear exceeds the dimension of the cochlear implant electrode array by at least a threshold amount. The marginal clearance region 514 can correspond to insertion angles where the cross-sectional area of the ear approaches or is less than the dimension of the cochlear implant electrode array. As shown in FIG. 5, the adequate clearance region 512 can span the lower insertion angles and the marginal clearance region 514 can span the higher insertion angles. A boundary between the adequate clearance region 512 and the marginal clearance region 514 can be indicated by a vertical dashed line at a transition insertion angle, such as at approximately 250°.

The graphical representation can display electrophysiologic responses at multiple frequencies during both insertion and withdrawal of the cochlear implant electrode array. As shown in FIG. 5, the multiple frequencies can include 1000 Hz, 500 Hz, and 250 Hz. Each frequency can be displayed using a distinct marker shape and a distinct line style. For example, the 1000 Hz responses can be displayed using square markers with a solid line, the 500 Hz responses can be displayed using triangle markers with a dashed line, and the 250 Hz responses can be displayed using diamond markers with a dotted line.

Insertion-phase responses and withdrawal-phase responses can be visually distinguished using filled and open markers, respectively. For example, the insertion-phase responses can be displayed using filled markers (filled squares for 1000 Hz, filled triangles for 500 Hz, filled diamonds for 250 Hz) and the withdrawal-phase responses can be displayed using open markers (open squares for 1000 Hz, open triangles for 500 Hz, open diamonds for 250 Hz). A legend can be included in the graphical representation to identify the marker shape, line style, and fill corresponding to each frequency and phase. The insertion direction indicator 516 can indicate the direction of electrode advancement toward increasing insertion angles, and the withdrawal direction indicator 518 can indicate the direction of electrode withdrawal toward decreasing insertion angles.

The insertion-phase responses can remain displayed at the insertion angles at which the responses were recorded after the electrode array has been withdrawn past those positions. The withdrawal-phase responses can be plotted at the insertion angles at which they were sampled during withdrawal. As shown in FIG. 5, the insertion-phase responses (filled markers) can span from 0° to approximately 350°, and the withdrawal-phase responses (open markers) can span from approximately 350° back to approximately 250°. Retaining both the insertion-phase responses and the withdrawal-phase responses on the same graphical representation can permit a clinician to compare electrophysiologic responses recorded at the same insertion angle during different phases of the procedure.

The current position indicator 214 can indicate the present location of the cochlear implant electrode array within the ear of the patient. As shown in FIG. 5, the current position indicator 214 can be displayed as a cross marker at approximately 200°, indicating that the electrode array has been withdrawn from a deeper insertion angle to the 200° position. The past position indicators 216 can indicate positions along the insertion angle at which prior electrophysiologic responses were sampled during both the insertion phase and the withdrawal phase. The current position indicator 214 can be visually distinguished from the past position indicators 216.

The controller circuit 122 (FIG. 1) can continue to receive electrophysiologic responses and position data during withdrawal. The controller circuit 122 can update the graphical representation by moving the current position indicator 214, adding withdrawal-phase responses at the current insertion angle, and retaining the insertion-phase responses at the previously recorded insertion angles. The adequate clearance region 512, the marginal clearance region 514, and the cochlear model 206 can remain displayed during withdrawal.

The controller circuit 122 can compare insertion-phase responses and withdrawal-phase responses at overlapping insertion angles. A difference between an electroacoustic response recorded during insertion and an electroacoustic response recorded during withdrawal at the same insertion angle can indicate a change in the condition of the cochlea at that location. The controller circuit 122 can display an indicator of the difference on the graphical representation or can generate an alert based on the difference.

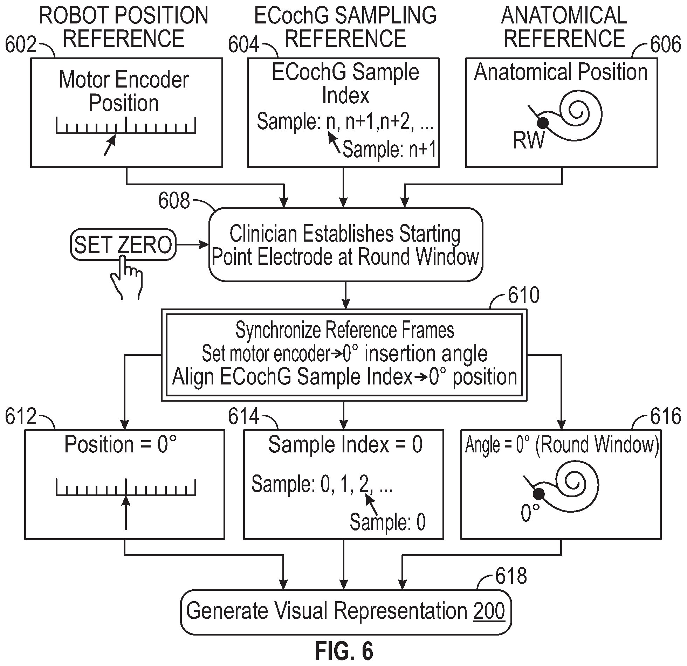

FIG. 6 illustrates a diagram of an example of a synchronization process 600 for aligning reference frames for intraoperative graphical feedback during a cochlear implant procedure. The synchronization process 600 can align three independent reference frames to a common starting point before or during insertion of the cochlear implant electrode array.

The three reference frames can include a robot position reference 602, an ECochG sampling reference 604, and an anatomical reference 606. The robot position reference 602 can represent a motor encoder position of the implant-positioning unit 110 (FIG. 1). Prior to synchronization, the motor encoder position can be at an arbitrary value that does not correspond to a known anatomical location within the ear of the patient. The ECochG sampling reference 604 can represent an index of electroacoustic response samples collected by the sensor. Prior to synchronization, the ECochG sample index can be at an arbitrary value (e.g., sample n, n+1, n+2) that does not correspond to a known insertion angle or anatomical position. The anatomical reference 606 can represent the physical anatomy of the ear of the patient, including the round window (RW) of the cochlea.

At step 608, a clinician can establish a starting point by positioning the cochlear implant electrode array at the round window and providing a user input to the system. As shown in FIG. 6, the user input can be a "SET ZERO" command provided via the user interface module 121 (FIG. 1), such as a button press, a touch screen input, or a voice command.

In response to the user input, the controller circuit 122 (FIG. 1) can synchronize the three reference frames at step 610. As shown in FIG. 6, the synchronization at step 610 can include setting the motor encoder to correspond to 0° insertion angle and aligning the ECochG sample index to correspond to the 0° position. The synchronization can map the motor encoder position at the time of the user input to the 0° insertion angle, such that subsequent motor encoder positions can be converted to insertion angles relative to the round window. The synchronization can also designate the ECochG sample index at the time of the user input as the zero-index sample, such that subsequent samples can be associated with insertion angles based on their offset from the zero-index sample. The relationship between the pre-synchronization offsets of the three reference frames can be as described with respect to FIG. 4.

Following synchronization, the robot position reference can be set to Position = 0° (612), the ECochG sampling reference can be set to Sample Index = 0 (614), and the anatomical reference can be set to Angle = 0° at the round window (616). At step 618, the controller circuit 122 can generate the visual representation 200 (FIG. 2) as described with respect to FIG. 5. Because the three reference frames can be synchronized to a common starting point, the controller circuit 122 can map each subsequent motor encoder position to a corresponding insertion angle, associate each subsequent ECochG sample with the insertion angle at which it was collected, and overlay the electrophysiologic responses and the position data onto the cochlear model 206 (FIG. 2) at the corresponding anatomical locations.

In examples, electroacoustic response samples collected before the synchronization event at step 608 can be discarded, or can be retained and flagged as pre-synchronization data as described with respect to FIG. 4. Pre-synchronization data can be excluded from the visual representation 200 or can be displayed with a visual indicator distinguishing it from post-synchronization data.

In examples, the synchronization process 600 can be repeated during a procedure. For example, if the cochlear implant electrode array is fully withdrawn and repositioned at the round window, the clinician can provide a second user input to re-synchronize the reference frames.

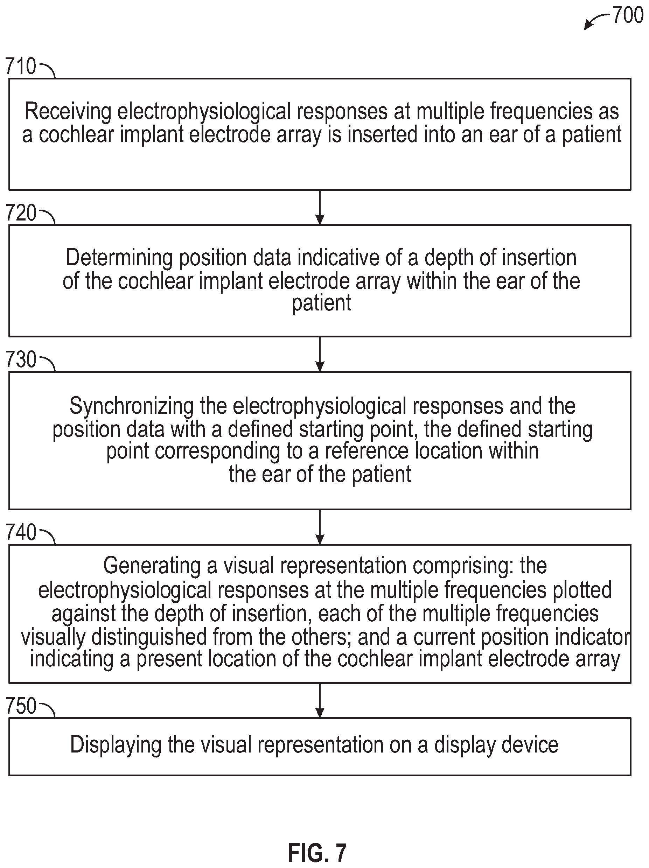

FIG. 7 illustrates a block diagram of an example of a method 700 for monitoring cochlear implant insertion. The method 700 can be performed by the controller circuit 122 (FIG. 1) of the external control console 120 (FIG. 1), by the processor 302 (FIG. 3) of the machine 300 (FIG. 3), or by another processor executing instructions stored in a memory. The method 700 can generate the visual representation 200 (FIG. 2) as described with respect to FIG. 5. The operations of the method 700 can be performed in the order shown or in a different order, and two or more operations can be performed concurrently.

At operation 710, the method 700 can include receiving, from a sensor, electrophysiologic responses at multiple frequencies as a cochlear implant electrode array is inserted into an ear of a patient. The sensor can be an electrocochleography (ECochG) sensor included in the sensor circuit 112 (FIG. 1), and the real-time electrophysiologic responses can include ECochG responses such as cochlear microphonic responses, summating potential responses, compound action potential responses, or a combination thereof. The acoustic stimuli at the multiple frequencies can be delivered by an acoustic stimulus source. The multiple frequencies can include at least a first frequency, a second frequency, and a third frequency, such as 1000 Hz, 500 Hz, and 250 Hz. Each of the multiple frequencies can be received as a distinct response signal from the sensor.

At operation 720, the method 700 can include determining position data indicative of a depth of insertion of the cochlear implant electrode array within the ear of the patient. The position data can be determined using a position sensor, such as the sensor circuit 112. In examples, the position data can be calculated based on a known insertion rate and an elapsed time since a reference point. The position data can be indicative of an insertion angle of the cochlear implant electrode array within the cochlea.

At operation 730, the method 700 can include synchronizing the electrophysiologic responses and the position data with a defined starting point corresponding to an anatomical reference location within the ear of the patient. The synchronization can align the sensor, the position sensor, and the anatomical reference location to a common reference as described with respect to the synchronization process 600 (FIG. 6). The defined starting point can correspond to the round window of the cochlea.

Synchronizing at operation 730 can include receiving a user input indicating that the cochlear implant electrode array is at a known anatomical position, such as the round window. The user input can be a "SET ZERO" command provided via the user interface module 121 (FIG. 1) as described with respect to FIG. 6. Synchronizing at operation 730 can further include setting a reference position for the position sensor based on the user input, such as setting a motor encoder to correspond to 0° insertion angle. Synchronizing at operation 730 can further include aligning a sampling index of the sensor with the reference position, such as designating a current ECochG sample index as a zero-index sample. The pre-synchronization states of the robot position reference 602 (FIG. 6), the ECochG sampling reference 604 (FIG. 6), and the anatomical reference 606 (FIG. 6) can be as described with respect to FIG. 4.

At operation 740, the method 700 can include generating a visual representation. Operation 740 can include several sub-operations.

The method 700 can include obtaining a cochlear model corresponding to the anatomy of the ear of the patient. The cochlear model can be the cochlear model 206 (FIG. 2). Obtaining the cochlear model can include receiving pre-operative imaging data of the ear of the patient and generating the cochlear model based on the pre-operative imaging data. In examples, the cochlear model can be generated using intraoperative imaging, machine learning, or a combination thereof. The cochlear model can include information about tonotopic regions of the cochlea, and the visual representation can include a mapping of the electrophysiologic responses at the multiple frequencies to corresponding tonotopic regions. In examples, the method 700 can include calculating, using the cochlear model, a clearance value at positions along the depth of insertion. The clearance value can be based on a difference between a cross-sectional area of the ear and a dimension of the cochlear implant electrode array at corresponding positions.

The method 700 can include generating the visual representation to include the electrophysiologic responses at the multiple frequencies plotted against the depth of insertion. The depth of insertion can be represented as an insertion angle derived from the position data. Each of the multiple frequencies can be visually distinguished from the others. For example, the first frequency can be displayed using square markers with a solid line, the second frequency can be displayed using triangle markers with a dashed line, and the third frequency can be displayed using diamond markers with a dotted line, as shown in FIG. 5.

The visual representation can include the cochlear model with the electrophysiologic responses and a current position indicator overlaid on the cochlear model. The visual representation can include a clearance indicator representing the clearance value at positions along the insertion angle. The clearance indicator can include the adequate clearance region 512 (FIG. 5) and the marginal clearance region 514 (FIG. 5).

The visual representation can include the current position indicator indicating a present location of the cochlear implant electrode array, such as the current position indicator 214 (FIG. 2). The visual representation can further include past position indicators at positions where prior electrophysiologic responses were sampled, such as the past position indicators 216 (FIG. 2). The past position indicators can be visually distinguished from the current position indicator.

At operation 740, the method 700 can further include detecting a change in the electrophysiologic responses at one or more of the multiple frequencies that exceeds a threshold. The change can include a drop in electroacoustic response amplitude. The method 700 can include correlating the detected change with a position along the insertion angle and with the clearance value at that position. The method 700 can include displaying an indicator on the visual representation at the position where the change was detected. The indicator can be associated with the clearance value at that position, such as by displaying the indicator within or adjacent to the adequate clearance region 512 or the marginal clearance region 514 at the corresponding insertion angle.

At operation 740, the method 700 can further include updating the visual representation during withdrawal of the cochlear implant electrode array. During withdrawal, the method 700 can include updating the current position indicator while maintaining previously recorded electrophysiologic responses at their corresponding insertion angles on the visual representation. Withdrawal-phase responses can be visually distinguished from insertion-phase responses, such as by using open markers for withdrawal-phase responses and filled markers for insertion-phase responses as described with respect to FIG. 5.

At operation 750, the method 700 can include displaying the visual representation on a display device. The display device can be the output module 126 (FIG. 1) of the external control console 120 or the display unit 310 (FIG. 3) of the machine 300. The visual representation can be updated on the display device as the cochlear implant electrode array is inserted or withdrawn.

In examples, the method 700 can further include controlling a robotic insertion mechanism based on the electrophysiologic responses and the position data. The robotic insertion mechanism can be the implant-positioning unit 110 (FIG. 1). Controlling the robotic insertion mechanism can include adjusting an insertion speed or trajectory based on the electrophysiologic responses, the position data, the clearance value, or a combination thereof. For example, the method 700 can include reducing the insertion speed when a drop in electroacoustic response is detected, pausing the insertion when the clearance value falls below a threshold, or reversing the insertion when a pattern indicative of potential trauma is detected.

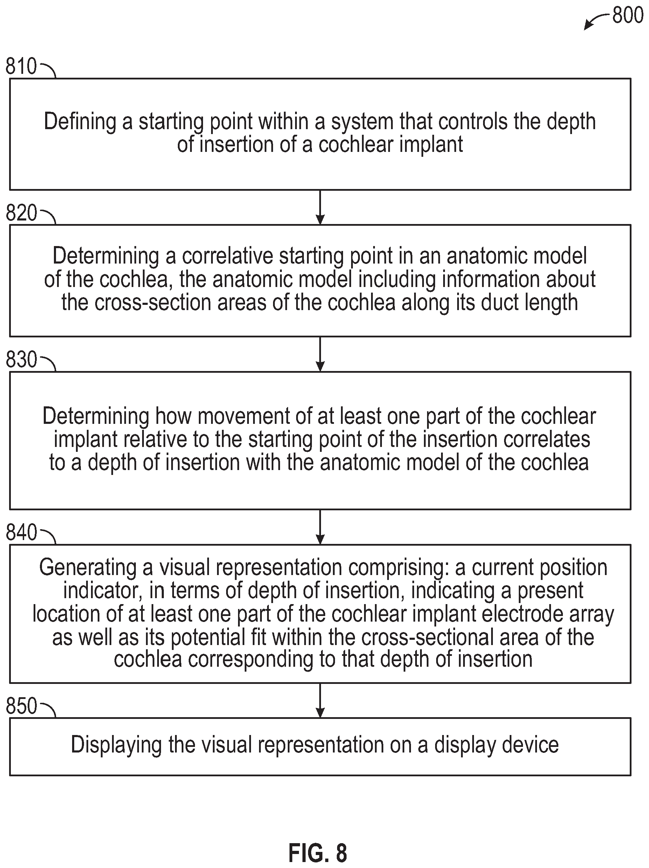

FIG. 8 illustrates a method 800 for monitoring cochlear implant insertion. The method 800 can be performed by a system including a processor executing instructions stored in a memory, such as the controller circuit 122 of the external control console 120 described with respect to FIG. 1, or the processor 302 of the machine 300 described with respect to FIG. 3.

At operation 810, the system can define a starting point within a system that controls the depth of insertion of a cochlear implant. The starting point can establish a reference position for tracking subsequent movement of the cochlear implant electrode array during the insertion procedure. In examples, defining the starting point can comprise receiving a user input indicating that the cochlear implant electrode array is at a known anatomical position within the ear of the patient. For example, a clinician can position the cochlear implant electrode array at the round window opening of the cochlea and can provide an input to the system indicating that the electrode array is at that position. The system can record the position of the cochlear implant electrode array as reported by a position sensor, a robotic insertion mechanism, or both, at the time the user input is received, and can set that recorded position as the starting point. In examples, the starting point can correspond to a position other than the round window opening, such as the entrance to the scala tympani or another anatomical landmark selected by the clinician. The starting point can also be defined based on a sensor signal, such as an impedance change detected at the electrode array indicating contact with a known anatomical structure.

At operation 820, the system can determine a correlative starting point in an anatomic model of the cochlea of the patient. The anatomic model can include information about cross-sectional areas of the cochlea along the duct length of the cochlea. In examples, the anatomic model can be generated from pre-operative imaging data of the ear of the patient, such as computed tomography (CT) scans or magnetic resonance imaging (MRI) data. The anatomic model can include measurements of the scala tympani height at positions along the cochlear duct, expressed as a function of insertion angle or duct length. The correlative starting point in the anatomic model can correspond to the anatomical position established at operation 810. For example, if the clinician defined the starting point as the round window opening at operation 810, the system can identify the corresponding position in the anatomic model that represents the round window opening. This correlation between the physical starting point and the model starting point can allow the system to map subsequent physical movement of the cochlear implant electrode array to corresponding positions within the anatomic model.

At operation 830, the system can determine how movement of at least one part of the cochlear implant relative to the starting point of the insertion correlates to a depth of insertion within the anatomic model of the cochlea. As the robotic insertion mechanism advances the cochlear implant electrode array, the system can track the distance traveled by the electrode array from the starting point defined at operation 810. The system can correlate this measured insertion distance to a corresponding insertion angle or duct length position within the anatomic model using the correlative starting point established at operation 820. In examples, the correlation can account for the geometry of the cochlear duct. Because the cochlea is a spiral structure, a linear insertion distance measured at the coupling unit may not correspond to a uniform angular displacement within the cochlea. The anatomic model can include a mapping between linear insertion distance and insertion angle that accounts for the spiral geometry of the cochlea of the patient. In examples, the system can determine the correlation in real time as the insertion progresses, continuously mapping the current insertion distance to the corresponding position in the anatomic model.

At operation 840, the system can generate a visual representation comprising a current position indicator indicating, in terms of the depth of insertion, a present location of at least one part of the cochlear implant electrode array, as well as the fit of that part of the cochlear implant electrode array within the cross-sectional area of the cochlea corresponding to that depth of insertion. The current position indicator can comprise a graphical representation of the tip of the cochlear implant electrode array scaled to match the scala tympani height at the current depth of insertion. The position of the graphical representation of the tip along a horizontal axis of the visual representation can be determined by the insertion distance relative to the starting point, correlated to the insertion angle via the anatomic model as described at operation 830.

The visual representation can further include representations of scala tympani height along sections of the cochlea corresponding to the depth of insertion. These representations can be displayed as color-coded regions. For example, a first color can indicate sections of the cochlea where the scala tympani height is larger than a dimension of the tip of the cochlear implant electrode array, and a second color can indicate sections where the scala tympani height is smaller than the dimension of the tip. This color coding can allow the clinician to see, at each position along the insertion path, whether the cochlear implant electrode array has sufficient space within the scala tympani or whether the electrode array may contact the basilar membrane or other cochlear structures due to insufficient clearance.

The visual representation can further include a pre-operative audiogram of the patient aligned along an axis of the visual representation according to a tonotopic map between cochlear duct depth and cochlear frequency response. The tonotopic map can correlate positions along the cochlear duct to the sound frequencies to which those positions are sensitive. By aligning the audiogram along the same axis as the insertion depth, the visual representation can allow the clinician to see the relationship between the electrode position, the available clearance, and the patient's residual hearing at each region of the cochlea. For example, the clinician can observe that the electrode array is approaching a region where the patient retains hearing function and where the available clearance is decreasing, and can use that information to make decisions about the insertion.

The visual representation can be updated in real time as the cochlear implant electrode array is advanced or withdrawn. As the electrode array moves, the current position indicator can update to reflect the present position, and any previously generated data (such as prior position indicators or previously recorded electroacoustic responses, if available) can remain displayed at their corresponding positions.

At operation 850, the system can display the visual representation on a display device. The display device can be the display unit 310 described with respect to FIG. 3, the output module 126 described with respect to FIG. 1, or another display device communicatively coupled to the system. The visual representation can be displayed in real time during the insertion procedure, providing the clinician with continuous intraoperative feedback.

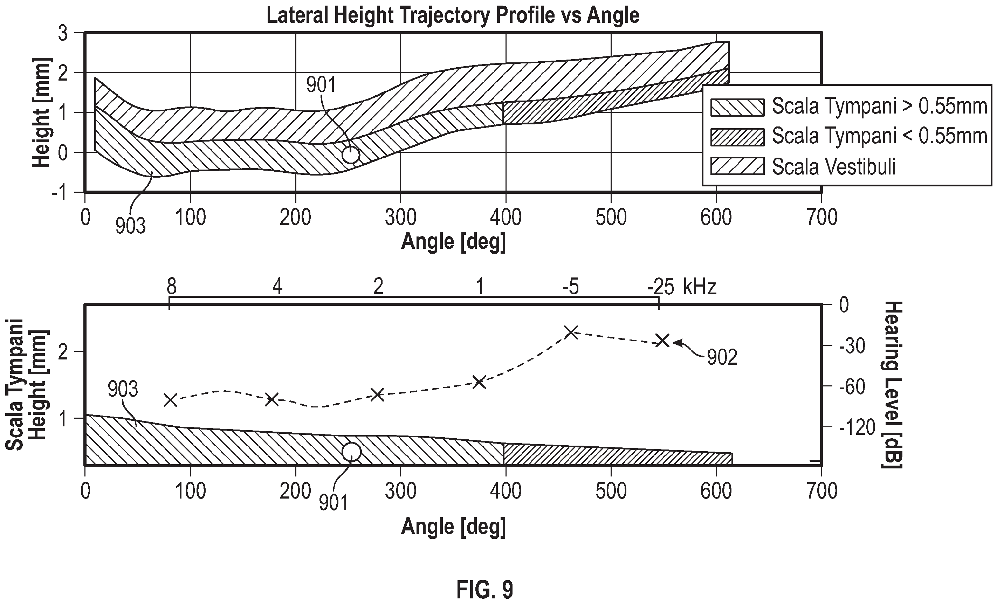

FIG. 9 illustrates an example of a visual representation 900 that can be generated by the system during a cochlear implant insertion procedure. The visual representation 900 can be generated at operation 840 and displayed at operation 850 of the method 800 described with respect to FIG. 8. The visual representation 900 can be displayed on the output module 126 described with respect to FIG. 1 or the display unit 310 described with respect to FIG. 3.

The visual representation 900 can include an electrode tip representation 901, a pre-operative audiogram 902, and scala tympani height representations 903.

The electrode tip representation 901 can be a graphical representation of the tip of the cochlear implant electrode array, scaled to match the scala tympani height at the current depth of insertion. The scaling of the electrode tip representation 901 can allow the clinician to visually assess the fit of the electrode array within the scala tympani at the present insertion depth. The position of the electrode tip representation 901 along the horizontal axis of the visual representation 900 can be determined by measuring the insertion distance of the cochlear implant electrode array relative to the defined starting point of robotic insertion, as established at operation 810 of the method 800. The measured insertion distance can be correlated to a corresponding insertion angle using the anatomic model established from pre-operative imaging and analysis of those images, as described at operations 820 and 830 of the method 800. As the cochlear implant electrode array is advanced or withdrawn during the insertion procedure, the electrode tip representation 901 can move along the horizontal axis to reflect the current position of the electrode array within the cochlea.

The pre-operative audiogram 902 can be a representation of the patient's pre-operative hearing test results aligned along the horizontal axis of the visual representation 900 according to a predetermined tonotopic map between cochlear duct depth and cochlear frequency response. The tonotopic map can correlate positions along the length of the cochlear duct, expressed as insertion angle or duct length, to the sound frequencies to which those positions of the cochlea are sensitive. By aligning the audiogram 902 along the same horizontal axis as the electrode tip representation 901 and the scala tympani height representations 903, the visual representation 900 can present the patient's residual hearing function in spatial registration with the electrode position and the available clearance within the cochlea. This alignment can allow the clinician to identify regions of the cochlea where the patient retains hearing function and to assess the proximity of the electrode array to those regions during the insertion. For example, the clinician can observe that the electrode array is approaching a tonotopic region where the audiogram 902 indicates residual low-frequency hearing, and can use that information in conjunction with the scala tympani height representations 903 to make decisions about whether to continue, slow, or halt the insertion.

The scala tympani height representations 903 can be graphical representations of the scala tympani height along sections of the cochlea corresponding to the insertion angle. The scala tympani height at each section can be derived from the anatomic model of the cochlea described at operation 820 of the method 800. The scala tympani height representations 903 can be displayed as color-coded regions on the visual representation 900. A first color, such as green, can indicate sections of the cochlea where the scala tympani height is larger than a dimension of the tip of the cochlear implant electrode array. In these sections, the electrode array can have clearance within the scala tympani and may not contact the basilar membrane or other cochlear structures. A second color, such as yellow, can indicate sections of the cochlea where the scala tympani height is smaller than the dimension of the tip of the cochlear implant electrode array. In these sections, the electrode array may impinge on the basilar membrane because the available space within the scala tympani may be insufficient to accommodate the electrode tip without contact. The boundary between the first color and the second color can correspond to the position along the insertion angle at which the scala tympani height transitions from being larger than the electrode tip dimension to being smaller than the electrode tip dimension.

The combination of the electrode tip representation 901, the pre-operative audiogram 902, and the scala tympani height representations 903 on a common horizontal axis referenced to the insertion angle can provide the clinician with a single visual representation 900 that simultaneously presents the current electrode position, the mechanical fit of the electrode within the cochlea, the regions of potential contact between the electrode and the basilar membrane, and the patient's residual hearing function mapped to the corresponding cochlear regions. The visual representation 900 can be updated in real time during the insertion procedure as the electrode array is advanced or withdrawn, with the electrode tip representation 901 moving along the horizontal axis to reflect the current insertion depth while the scala tympani height representations 903 and the pre-operative audiogram 902 can remain in their anatomically referenced positions.

The following, non-limiting examples, detail certain aspects of the present subject matter to solve the challenges and provide the benefits discussed herein, among others.

Example 1 is a system for monitoring cochlear implant insertion, comprising: a sensor configured to detect electrophysiologic responses at multiple frequencies; a memory including instructions; and a processor coupled to the memory, the processor including processing circuitry, the instructions, when executed by the processing circuitry, configure the processor to: receive, from the sensor, electrophysiologic responses; determine position data, the position data indicative of a depth of insertion of the cochlear implant electrode array within the ear of the patient; generate, using the electrophysiologic responses and the position data, a visual representation as the cochlear implant electrode array is inserted into the ear of the patient; and transmit the visual representation to a display, the visual representation including: the electrophysiologic responses at the multiple frequencies plotted against an insertion angle, the insertion angle indicative of the depth of insertion of the cochlear implant electrode array within the ear of the patient; and a current position indicator for the cochlear implant electrode array within the ear of the patient.

In Example 2, the subject matter of Example 1 optionally includes wherein the sensor includes an electrocochleography (ECochG) sensor, and wherein the electrophysiologic responses include ECochG responses.

In Example 3, the subject matter of Example 2 optionally includes wherein the instructions cause the processor to: obtain a cochlear model, the cochlear model based on the ear of the patient; wherein the visual representation overlays the ECochG responses at multiple frequencies plotted against the insertion angle and the current position indicator over the cochlear model.

In Example 4, the subject matter of Example 3 optionally includes wherein the instructions configure the processor to: determine, using the cochlear model, a cross-sectional area, the cross-sectional area corresponding to a diameter of the ear relative to the insertion angle.

In Example 5, the subject matter of Example 4 optionally includes wherein the instructions further configure the processor to: determine an available space by calculating a difference between a diameter of the cochlear implant electrode array and the cross-sectional area of the ear at corresponding insertion angles.

In Example 6, the subject matter of Example 5 optionally includes wherein the visual representation includes visual indicators configured to indicate the available space relative to insertion angles, and wherein generating the visual indicators comprises: assigning a first visual indicator to regions where the available space is greater than a threshold; assigning a second visual indicator to regions where the available space is less than the threshold but greater than the diameter of the cochlear implant electrode array; and assigning a third visual indicator to regions where the available space is less than or equal to the diameter of the cochlear implant electrode array.

In Example 7, the subject matter of any one or more of Examples 3-6 optionally include wherein the instructions further configure the processor to: correlate changes in the ECochG responses with specific locations in the cochlear model; and include indicators of a correlation in the visual representation.

In Example 8, the subject matter of any one or more of Examples 3-7 optionally include wherein the cochlear model includes information about tonotopic regions of a cochlea of the patient, and wherein the visual representation includes a mapping of the ECochG responses to corresponding tonotopic regions.

In Example 9, the subject matter of any one or more of Examples 3-8 optionally include wherein obtaining the cochlear model comprises: receiving pre-operative imaging data of the ear of the patient; and generating the cochlear model based on the pre-operative imaging data.

In Example 10, the subject matter of any one or more of Examples 2-9 optionally include wherein the instructions further configure the processor to: update the visual representation in as the cochlear implant electrode array is inserted, including updating the current position indicator and plotted ECochG responses.

In Example 11, the subject matter of any one or more of Examples 2-10 optionally include wherein the instructions further configure the processor to: detect drops in ECochG response; correlate the drops with specific locations along an insertion path of the cochlear implant electrode array; and include indicators of a correlated drops in the visual representation.

In Example 12, the subject matter of any one or more of Examples 2-11 optionally include a robotic insertion mechanism, wherein the instructions further configure the processor to: control the robotic insertion mechanism based on the ECochG responses and the position data.

In Example 13, the subject matter of any one or more of Examples 1-12 optionally include wherein the instructions further configure the processor to: synchronize the electrophysiologic responses and the position data with a defined starting point of the cochlear implant insertion.

In Example 14, the subject matter of any one or more of Examples 1-13 optionally include wherein the visual representation is configured to provide continuous positional feedback relative to anatomy of the ear during both insertion and withdrawal to accommodate reversible insertions of the cochlear implant electrode array.

In Example 15, the subject matter of any one or more of Examples 3-14 optionally include wherein the instructions further configure the processor to: update the cochlear model in based on the ECochG responses and the position data.

In Example 16, the subject matter of Example 15 optionally includes wherein the instructions further configure the processor to: generate a multi-factor analysis based on the ECochG responses, the position data, and the cochlear model; and include the multi-factor analysis in the visual representation.

In Example 17, the subject matter of any one or more of Examples 1-16 optionally include wherein the visual representation further includes: a graphical indicator of insertion progress relative to predefined anatomical landmarks within a cochlea of a patient.

In Example 18, the subject matter of any one or more of Examples 1-17 optionally include a robotic insertion mechanism, wherein the instructions further configure the processor to: control the robotic insertion mechanism based on the electrophysiologic responses and the position data.

In Example 19, the subject matter of Example 18 optionally includes wherein the instructions further configure the processor to: adjust an insertion speed or trajectory of the robotic insertion mechanism based on the electrophysiologic responses and the position data.

In Example 20, the subject matter of any one or more of Examples 1-19 optionally include wherein the instructions further configure the processor to: detect patterns in the electrophysiologic responses indicative of potential trauma during insertion; and generate an alert signal when a pattern indicative of potential trauma is detected.

Example 21 is a system for monitoring cochlear implant insertion, comprising: a sensor configured to detect electrophysiologic responses at multiple frequencies during insertion of a cochlear implant electrode array into an ear of a patient; a memory storing a cochlear model corresponding to anatomy of the ear of the patient and storing instructions; and processing circuitry coupled to the memory, the instructions, when executed by the processing circuitry, configure the processing circuitry to: receive, from the sensor, electrophysiologic responses at the multiple frequencies; determine position data indicative of a depth of insertion of the cochlear implant electrode array within the ear of the patient; synchronize the electrophysiologic responses and the position data with a defined starting point, the defined starting point corresponding to an anatomical reference location within the ear of the patient; determine, using the cochlear model, a cross-sectional dimension of the ear at positions corresponding to the depth of insertion; calculate a clearance value at each of the positions based on a difference between the cross-sectional dimension of the ear and a corresponding dimension of the cochlear implant electrode array; generate a visual representation comprising: the electrophysiologic responses at the multiple frequencies plotted against the depth of insertion, each of the multiple frequencies visually distinguished from the others; the cochlear model overlaid with the plotted electrophysiologic responses; a clearance indicator representing the clearance value at positions along the depth of insertion; and a current position indicator indicating a present location of the cochlear implant electrode array within the ear of the patient; and transmit the visual representation to a display.

In Example 22, the subject matter of Example 21 optionally includes wherein the sensor comprises an electrocochleography sensor, and wherein the electrophysiologic responses comprise electrocochleography responses.

In Example 23, the subject matter of any one or more of Examples 21-22 optionally include wherein the clearance indicator comprises a color-coded region overlaid on the visual representation, the color-coded region comprising: a first color corresponding to positions where the clearance value exceeds a first threshold; a second color corresponding to positions where the clearance value is between the first threshold and a second threshold; and a third color corresponding to positions where the clearance value is at or below the second threshold.

In Example 24, the subject matter of Example 23 optionally includes wherein the second threshold corresponds to a condition where the corresponding dimension of the cochlear implant electrode array is equal to or greater than the cross-sectional dimension of the ear.

In Example 25, the subject matter of any one or more of Examples 21-24 optionally include wherein the defined starting point corresponds to a round window opening of a cochlea of the patient, and wherein synchronizing comprises receiving a user input indicating that the cochlear implant electrode array is positioned at the round window opening.

In Example 26, the subject matter of any one or more of Examples 21-25 optionally include wherein the visual representation further comprises past position indicators at positions where prior electrophysiologic responses were sampled, the past position indicators visually distinguished from the current position indicator.

In Example 27, the subject matter of any one or more of Examples 21-26 optionally include wherein the instructions further configure the processing circuitry to: update the visual representation during both insertion and withdrawal of the cochlear implant electrode array, including updating the current position indicator during withdrawal while continuing to display previously recorded electrophysiologic responses at their corresponding positions along the depth of insertion.

In Example 28, the subject matter of any one or more of Examples 21-27 optionally include wherein the cochlear model comprises tonotopic region data mapping frequency sensitivity to positions along a length of a cochlea of the patient, and wherein the visual representation maps the electrophysiologic responses at the multiple frequencies to corresponding tonotopic regions.

In Example 29, the subject matter of any one or more of Examples 21-28 optionally include wherein the position data is determined based on one or more of: a signal from a position sensor, an optical sensor signal, or a calculation based on a known insertion rate and an elapsed time.

Example 30 is a method for monitoring cochlear implant insertion, comprising: receiving, from a sensor, electrophysiologic responses at multiple frequencies as a cochlear implant electrode array is inserted into an ear of a patient; determining position data indicative of a depth of insertion of the cochlear implant electrode array within the ear of the patient; synchronizing the electrophysiologic responses and the position data with a defined starting point, the defined starting point corresponding to a reference location within the ear of the patient; generating a visual representation comprising: the electrophysiologic responses at the multiple frequencies plotted against the depth of insertion, each of the multiple frequencies visually distinguished from the others; and a current position indicator indicating a present location of the cochlear implant electrode array; and displaying the visual representation on a display device.

In Example 31, the subject matter of Example 30 optionally includes wherein synchronizing comprises: receiving a user input indicating that the cochlear implant electrode array is at the reference location; setting a reference for the position data based on the user input; and associating the electrophysiologic responses received after the user input with corresponding values of the position data relative to the reference.

In Example 32, the subject matter of any one or more of Examples 30-31 optionally include obtaining a cochlear model corresponding to anatomy of the ear of the patient; and overlaying the plotted electrophysiologic responses and the current position indicator on the cochlear model in the visual representation.

In Example 33, the subject matter of Example 32 optionally includes calculating, using the cochlear model, a clearance value at positions along the depth of insertion, the clearance value based on a difference between a cross-sectional dimension of the ear and a corresponding dimension of the cochlear implant electrode array at each of the positions; and displaying a clearance indicator representing the clearance value at positions along the depth of insertion in the visual representation.

In Example 34, the subject matter of Example 33 optionally includes detecting a change in the electrophysiologic responses at one or more of the multiple frequencies that exceeds a threshold; correlating the change with a position along the depth of insertion and with the clearance value at the position; and displaying an indicator on the visual representation at the position where the change was detected.

In Example 35, the subject matter of any one or more of Examples 30-34 optionally include during withdrawal of the cochlear implant electrode array, updating the current position indicator while continuing to display previously recorded electrophysiologic responses at their corresponding positions along the depth of insertion.

In Example 36, the subject matter of any one or more of Examples 30-35 optionally include wherein the multiple frequencies comprise at least a first frequency, a second frequency, and a third frequency, and wherein the visual representation displays each of the first frequency, the second frequency, and the third frequency with a visually distinct marker type, color, or both.

Example 37 is a computer-readable medium storing instructions that, when executed by processing circuitry, cause the processing circuitry to: receive, from a sensor, electrophysiologic responses at multiple frequencies during insertion of a cochlear implant electrode array into an ear of a patient; determine position data indicative of a depth of insertion of the cochlear implant electrode array within the ear of the patient; synchronize the electrophysiologic responses and the position data with a defined starting point corresponding to an anatomical reference location within the ear of the patient; obtain a cochlear model corresponding to anatomy of the ear of the patient; calculate, using the cochlear model, a clearance value at positions along the depth of insertion based on a difference between a cross-sectional dimension of the ear and a corresponding dimension of the cochlear implant electrode array; generate a visual representation comprising: the electrophysiologic responses at the multiple frequencies plotted against the depth of insertion, the electrophysiologic responses overlaid on the cochlear model; a clearance indicator representing the clearance value at positions along the depth of insertion; and a current position indicator indicating a present location of the cochlear implant electrode array; and transmit the visual representation to a display.

In Example 38, the subject matter of Example 37 optionally includes wherein the instructions further cause the processing circuitry to: receive a user input establishing the defined starting point, the user input indicating that the cochlear implant electrode array is at the anatomical reference location; and set a reference for the position data and the electrophysiologic responses based on the user input.

In Example 39, the subject matter of any one or more of Examples 37-38 optionally include wherein the instructions further cause the processing circuitry to: detect a drop in the electrophysiologic responses at one or more of the multiple frequencies; correlate the drop with the clearance value at a corresponding position along the depth of insertion; and display a correlation indicator on the visual representation at the corresponding position.

In Example 40, the subject matter of any one or more of Examples 37-39 optionally include wherein the instructions further cause the processing circuitry to: update the visual representation during withdrawal of the cochlear implant electrode array, including moving the current position indicator in a withdrawal direction while continuing to display previously recorded electrophysiologic responses at their corresponding positions along the depth of insertion.

Example 41 is a method for monitoring cochlear implant insertion, comprising: defining, within a system that controls a depth of insertion of a cochlear implant, a starting point for the insertion; determining a correlative starting point in an anatomic model of a cochlea of a patient, the anatomic model including information about cross-sectional areas of the cochlea along a duct length of the cochlea; determining how movement of at least one part of the cochlear implant relative to the starting point correlates to a depth of insertion within the anatomic model of the cochlea; generating a visual representation comprising a current position indicator indicating, in terms of the depth of insertion, a present location of at least one part of a cochlear implant electrode array and a fit of the at least one part of the cochlear implant electrode array within a cross-sectional area of the cochlea corresponding to the depth of insertion; and displaying the visual representation on a display device.

In Example 42, the subject matter of Example 41 optionally includes wherein the current position indicator comprises a graphical representation of a tip of the cochlear implant electrode array scaled to match a scala tympani height at the depth of insertion.

In Example 43, the subject matter of Example 42 optionally includes wherein a position of the graphical representation of the tip along a horizontal axis of the visual representation is determined by measuring an insertion distance of the cochlear implant electrode array relative to the starting point and correlating the insertion distance to an insertion angle using the anatomic model.

In Example 44, the subject matter of any one or more of Examples 41-43 optionally include wherein the visual representation further comprises representations of scala tympani height along sections of the cochlea corresponding to the depth of insertion.

In Example 45, the subject matter of Example 44 optionally includes wherein the representations of scala tympani height comprise color-coded regions, a first color indicating sections where the scala tympani height is larger than a dimension of the tip of the cochlear implant electrode array, and a second color indicating sections where the scala tympani height is smaller than the dimension of the tip of the cochlear implant electrode array.

In Example 46, the subject matter of any one or more of Examples 41-45 optionally include wherein the visual representation further comprises a pre-operative audiogram of the patient aligned along an axis of the visual representation according to a tonotopic map between cochlear duct depth and cochlear frequency response.

In Example 47, the subject matter of Example 46 optionally includes wherein the tonotopic map correlates the depth of insertion to corresponding frequency regions of the cochlea, and wherein the pre-operative audiogram is aligned such that audiometric frequencies correspond to insertion angles associated with those frequency regions.

In Example 48, the subject matter of any one or more of Examples 41-47 optionally include wherein the anatomic model is generated from pre-operative imaging data of the ear of the patient.

In Example 49, the subject matter of any one or more of Examples 41-48 optionally include wherein defining the starting point comprises receiving a user input indicating that the cochlear implant electrode array is at a known anatomical position within the ear of the patient.

In Example 50, the subject matter of Example 49 optionally includes wherein the known anatomical position is a round window opening of the cochlea.

Example 51 includes a system, method, or device including any one or more elements of any one or more examples of Examples 1-50.

The above detailed description includes references to the accompanying drawings, which form a part of the detailed description. The drawings show, by way of illustration, specific examples that may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range (e.g., 1 to 5 includes 1, 1.5, 2, 2.75, 3, 3.90, 4, 4.24, and 5). Similarly, numerical ranges recited herein by endpoints include subranges subsumed within that range (e.g., 1 to 5 includes 1-1.5, 1.5-2, 2-2.75, 2.75-3, 3-3.90, 3.90-4, 4-4.24, 4.24-5, 2-5, 3-5, 1-4, and 2-4). It is also to be understood that all numbers and fractions thereof are presumed to be modified by the term "about."

The above description is intended to be illustrative, and not restrictive. For example, the above-described examples (or one or more aspects thereof) may be used in combination with each other. Other examples may be used, such as by one of ordinary skill in the art upon reviewing the above description. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the examples should be determined with reference to the appended claims.

## Claims

1. A system for monitoring cochlear implant insertion, comprising:
a sensor configured to detect electrophysiologic responses at multiple frequencies during insertion of a cochlear implant electrode array into an ear of a patient;
a memory storing a cochlear model corresponding to anatomy of the ear of the patient and storing instructions; and
processing circuitry coupled to the memory, the instructions, when executed by the processing circuitry, configure the processing circuitry to:
receive, from the sensor, electrophysiologic responses at the multiple frequencies;
determine position data indicative of a depth of insertion of the cochlear implant electrode array within the ear of the patient;
synchronize the electrophysiologic responses and the position data with a defined starting point, the defined starting point corresponding to an anatomical reference location within the ear of the patient;
determine, using the cochlear model, a cross-sectional dimension of the ear at positions corresponding to the depth of insertion;
calculate a clearance value at each of the positions based on a difference between the cross-sectional dimension of the ear and a corresponding dimension of the cochlear implant electrode array;
generate a visual representation comprising:
the electrophysiologic responses at the multiple frequencies plotted against the depth of insertion, each of the multiple frequencies visually distinguished from the others;
the cochlear model overlaid with the plotted electrophysiologic responses;
a clearance indicator representing the clearance value at positions along the depth of insertion; and
a current position indicator indicating a present location of the cochlear implant electrode array within the ear of the patient; and
transmit the visual representation to a display.

2. The system of claim 1, wherein the sensor comprises an electrocochleography sensor, and wherein the electrophysiologic responses comprise electrocochleography responses.

3. The system of claim 1 or claim 2, wherein the clearance indicator comprises a color-coded region overlaid on the visual representation, the color-coded region comprising:
a first color corresponding to positions where the clearance value exceeds a first threshold;
a second color corresponding to positions where the clearance value is between the first threshold and a second threshold; and
a third color corresponding to positions where the clearance value is at or below the second threshold,
wherein the second threshold optionally corresponds to a condition where the corresponding dimension of the cochlear implant electrode array is equal to or greater than the cross-sectional dimension of the ear.

4. The system of any one of claims 1 to 3, wherein the defined starting point corresponds to a round window opening of a cochlea of the patient, and wherein synchronizing comprises receiving a user input indicating that the cochlear implant electrode array is positioned at the round window opening.

5. The system of any one of claims 1 to 4, wherein the visual representation further comprises past position indicators at positions where prior electrophysiologic responses were sampled, the past position indicators visually distinguished from the current position indicator.

6. The system of any one of claims 1 to 5, wherein the instructions further configure the processing circuitry to:
update the visual representation during both insertion and withdrawal of the cochlear implant electrode array, including updating the current position indicator during withdrawal while continuing to display previously recorded electrophysiologic responses at their corresponding positions along the depth of insertion.

7. The system of any one of claims 1 to 6, wherein the cochlear model comprises tonotopic region data mapping frequency sensitivity to positions along a length of a cochlea of the patient, and wherein the visual representation maps the electrophysiologic responses at the multiple frequencies to corresponding tonotopic regions.

8. The system of any one of claims 1 to 7, wherein the position data is determined based on one or more of: a signal from a position sensor, an optical sensor signal, or a calculation based on a known insertion rate and an elapsed time.

9. A method for monitoring cochlear implant insertion, comprising:
receiving, from a sensor, electrophysiologic responses at multiple frequencies as a cochlear implant electrode array is inserted into an ear of a patient;
determining position data indicative of a depth of insertion of the cochlear implant electrode array within the ear of the patient;
synchronizing the electrophysiologic responses and the position data with a defined starting point, the defined starting point corresponding to a reference location within the ear of the patient;
generating a visual representation comprising:
the electrophysiologic responses at the multiple frequencies plotted against the depth of insertion, each of the multiple frequencies visually distinguished from the others; and
a current position indicator indicating a present location of the cochlear implant electrode array; and
displaying the visual representation on a display device.

10. The method of claim 9, wherein synchronizing comprises:
receiving a user input indicating that the cochlear implant electrode array is at the reference location;
setting a reference for the position data based on the user input; and
associating the electrophysiologic responses received after the user input with corresponding values of the position data relative to the reference.

11. The method of claim 9 or claim 10, further comprising:
obtaining a cochlear model corresponding to anatomy of the ear of the patient; and
overlaying the plotted electrophysiologic responses and the current position indicator on the cochlear model in the visual representation.

12. The method of claim 11, further comprising:
calculating, using the cochlear model, a clearance value at positions along the depth of insertion, the clearance value based on a difference between a cross-sectional dimension of the ear and a corresponding dimension of the cochlear implant electrode array at each of the positions; and
displaying a clearance indicator representing the clearance value at positions along the depth of insertion in the visual representation,
the method optionally further comprising:
detecting a change in the electrophysiologic responses at one or more of the multiple frequencies that exceeds a threshold;
correlating the change with a position along the depth of insertion and with the clearance value at the position; and
displaying an indicator on the visual representation at the position where the change was detected.

13. The method of any one of claims 9 to 12, further comprising:
during withdrawal of the cochlear implant electrode array, updating the current position indicator while continuing to display previously recorded electrophysiologic responses at their corresponding positions along the depth of insertion.

14. The method of any one of claims 9to 13, wherein the multiple frequencies comprise at least a first frequency, a second frequency, and a third frequency, and wherein the visual representation displays each of the first frequency, the second frequency, and the third frequency with a visually distinct marker type, color, or both.

15. A computer-readable medium storing instructions that, when executed by processing circuitry, cause the processing circuitry to perform the method of any one of claims 9 to 14.
